# EUROPEAN PATENT APPLICATION

(11) **EP 4 778 512 A2**
(43) Date of publication of application: **22.07.2026**
(21) Application number: 26177596.9
(22) Date of filing: 18.03.2024
(51) Int. Cl.: A61K 9/00

(54) **ORALLY DISINTEGRATING NICOTINE TABLET FOR USE UNDER LIP**

(30) Priority: 12.09.2023 DK PA202370471
(62) Divisional of application: 24713915.7
(71) Applicant: Fertin Pharma A/S, 7100 Vejle (DK)
(72) Inventor: NIELSEN, Kent Albin, 7100 Vejle (DK); NIELSEN, Bruno Provstgaard, 7100 Vejle (DK); MARTINUSSEN, Helle, 7100 Vejle (DK)
(74) Representative: Patentgruppen A/S

(57) **Abstract**

An orally disintegrating nicotine tablet for use in alleviation of nicotine craving is disclosed, the tablet comprising nicotine and a pH regulating agent, wherein the tablet is administered between the lip and gum. Also, a method for treatment of nicotine craving is disclosed.

## Description

### FIELD OF INVENTION

The invention relates to an orally disintegrating nicotine tablet for use in alleviation of nicotine craving according to the claims. Also, the invention relates to a method for treatment of nicotine craving according to the claims.

### BACKGROUND

Nicotine-releasing tablets applied for the purpose of providing a release of nicotine in a user's mouth over a certain period is well-known. Much effort has in prior art been put into emulating the nicotine release and oral perception of a cigarette when it is smoked by a user, which means that release profiles from nicotine tablets have been thoroughly investigated in prior art.

It is however an established fact that the one-to-one smoking-emulation is yet to be achieved with other means than a cigarette.

Furthermore, the release of nicotine is associated with undesirable irritation in the throat, a side effect known as nicotine burning or simply burning. Consequently, a trade off between releasing sufficient amount of nicotine and avoiding undesirable nicotine burning is sought.

An example of an orally disintegrating nicotine tablet is known from PCT application WO 2019/110072 A1, to Fertin Pharma A/S, related to orally disintegrating tablets with disintegration within 60 second from oral administration and suggesting the use as a sublingual tablet for positioning under the tongue, as a buccal tablet (i.e. positioning on the tongue).

### SUMMARY

The invention relates to an orally disintegrating nicotine tablet for use in alleviation of nicotine craving,
the tablet comprising nicotine and a pH regulating agent, wherein the tablet is administered between the lip and gum.

An advantage of the invention may be that fast nicotine craving relief may be obtained while minimizing any undesirable side effects from oral nicotine administration, in particular throat irritation, also commonly referred to as throat burning or simply burning.

Typically, oral nicotine release is associated with nicotine burning, an irritation of the mouth and in particular the throat. Therefore, a conventional consideration is that the release of nicotine should not be too fast in order to avoid swallowing of nicotine and thereby excessive nicotine burning.

The present inventor, however, surprisingly found that nicotine burning was minimized when using an orally disintegrating nicotine tablet administered between the lip and gum.

A further advantage of the invention may be that an improved nicotine effect may be obtained by using an orally disintegrating nicotine tablet administered between the lip and gum.

Also, the invention provides an effective delivery of nicotine. Since the orally disintegrating nicotine tablet completely dissolves, the entire content of nicotine is released orally.

Unexpectedly, the present inventors found that the tablet used in accordance with the invention led to a combination of significantly improved nicotine absorption while at the same time significantly reducing the undesirable nicotine burning side effect. Thereby, the inventive use facilitates a desirable user experience both in terms of fast and effective craving relief and in terms of a desirable mouthfeel, including decreased nicotine burning.

It is noted that in the present context, the term "orally disintegrating nicotine tablet" refers to a nicotine containing orally disintegrating tablet, also referred to as a nicotine ODT, i.e. a tablet that disintegrates relative fast. It is noted that orally disintegrating tablets, or ODTs, may sometimes be referred to as fast disintegrating tablets, or FDTs. In embodiments of the invention, the orally disintegrating nicotine tablet may thus disintegrate within 300 seconds upon oral administration between the lip and gum, such as within 270 seconds upon oral administration between the lip and gum, such as within 240 seconds upon oral administration between the lip and gum. In embodiments of the invention, the orally disintegrating nicotine tablet may disintegrate within 60 seconds in vitro, preferably when measuring vitro disintegration time in accordance with European Pharmacopoeia 9.0, section 2.9.1, Disintegration of tablets and capsules.

It is noted that the tablet is administered between the lip and gum, which is understood as positioning the tablet in the oral vestibule such that it is in contact with a part of the gum, also known as gingiva.

In an embodiment of the invention, the tablet is administered between the gum and the lip extending corresponding to the incisor, canine, premolar, and molar teeth. Thus, in this embodiment, the tablet would be positioned with one side of the tablet being in contact with the lip and the opposite side of the tablet being in contact with the gum opposite to the part of the lip in question.

In an embodiment of the invention, the tablet is administered between the gum and the lip, the latter extending corresponding to the incisor and canine teeth. Thus, in this embodiment, the tablet would be positioned with one side of the tablet being in contact with the lip above these teeth and the opposite side of the tablet being in contact with the gum opposite to the lip.

Particularly, the present inventors surprisingly found that improved nicotine uptake in the blood stream was demonstrated e.g. in terms of much higher maximum nicotine plasma concentration (Cmax) and area under the curve (AUC), when comparing to use on the tongue and sublingual use.

It is noted that by administering the tablet between the lip and gum is understood that the tablet is positioned in the oral vestibule such that it is in contact with a part of the gum and not removed, i.e. the ODT is allowed to disintegrate while remaining positioned in the oral vestibule.

It is noted that according to the invention, the tablet is administered between the lip and gum, i.e. that the tablet is to be administered between the lip and gum.

According to an advantageous embodiment of the invention, the tablet is administered between the upper lip and gum.

It is noted that in the context of the above embodiment, the position between the upper lip and the gum is the upper lip and the corresponding gum. Typically, the upper lip extends corresponding to the incisor and canine teeth and thus the tablet would be positioned with one side of the tablet being in contact with the upper lip above these teeth and the opposite side of the tablet being in contact with the gum opposite to the upper lip.

An advantage of the above embodiment may be that that the position provides a discreet method for delivering nicotine.

A further advantage of the above embodiment may be that that the position prevents swallowing of a tablet.

According to an advantageous embodiment of the invention, the tablet is designed to disintegrate within a period of 300 seconds upon oral administration between the lip and gum, such as within 270 seconds upon oral administration, such as within 240 seconds upon oral administration, such as within a period of 210 seconds upon oral administration, such as within a period of 180 seconds upon oral administration, such as within a period of 150 seconds upon oral administration, such as within a period of 120 seconds upon oral administration, such as within a period of 90 seconds upon oral administration, such as within a period of 60 seconds upon oral administration.

Thus, the above embodiment is directed to the in vivo disintegration time of the tablet when administered orally according to the invention, i.e. administered between the lip and gum. It is noted that the tablet is substantially completely disintegrated after 300 seconds, such as after 270 seconds, such as after 240 seconds. In an embodiment of the disintegration of the tablet is complete after 300 seconds, such as after 270 seconds, such as after 240 seconds.

In an embodiment of the invention, the tablet is designed to disintegrate within a period of 210 seconds upon oral administration, such as within a period of 180 seconds upon oral administration, such as within a period of 150 seconds upon oral administration, such as within a period of 120 seconds upon oral administration, such as within a period of 90 seconds upon oral administration, such as within a period of 60 seconds upon oral administration.

An advantage of the above embodiment may be that a fast craving relief of nicotine may be facilitated, e.g. due to a fast disintegration leading to a release of the nicotine comprised in a tablet.

According to an embodiment of the invention, the in vivo disintegration time is measured by at least 6 trained assessors, the trained assessors abstaining from eating and drinking at least 30 minutes before initiation of any test, the tablet is weighted and placed in the mouth, between the upper lip and the gum, where the in vivo disintegration time is registered as the time point where the tablet disintegration is substantially complete.

The trained assessors are selected based on the following criteria. First, the individuals chewed on a chewing gum base free of buffer for 1 minute, after which the initial pH in a sample from the saliva from each of the individuals is measured with a suitable pH-electrode system, e.g. a stainless steel electrode PHW77-SS. Only individuals having, after chewing on a chewing gum base free of buffer for one minute, an initial pH in the saliva inside the range from 6.7 and 7.3 are selected. These individuals thereby qualify as assessors. One dosage of the tablet is administered between the upper lip and the gum to at least six assessors. Hereafter, a desired sensory attribute (e.g. nicotine throat burning) from each of the six assessors is evaluated at specified time intervals. Thus, each parameter evaluation is the arithmetic mean of six assessments performed on samples from six assessors.

According to an embodiment of the invention, the tablet is designed to disintegrate within a period of 2 to 300 seconds upon oral administration, such as within a period of 3 to 270 seconds, such as within a period of 5 to 240 seconds upon oral administration, such as within a period of 10 and 180 seconds upon oral administration, such as 20 to 150 seconds, such as 30 to 120 seconds.

According to an advantageous embodiment of the invention, the tablet is designed to disintegrate within a period of 60 seconds in vitro, such as within 50 seconds in vitro, such as within 40 seconds in vitro.

It is noted that in vitro disintegration time are measured in accordance with European Pharmacopeia 9.0, section 2.9.1, Disintegration of tablets and capsules.

According to an embodiment of the invention, the tablet is designed to disintegrate within a period of 5 to 60 seconds in vitro, such as within a period of 10 to 50 seconds in vitro, such as within a period of 15 to 40 seconds in vitro.

According to an advantageous embodiment of the invention, the tablet is a compressed orally disintegrating nicotine tablet.

According to an advantageous embodiment of the invention, the tablet is composed of a plurality of compressed particles.

An advantage of the above embodiment may be that the tablet releases all its nicotine contents into the oral cavity.

A further advantage of the above embodiment may be that it is easier to produce a tablet containing a specific dose. Thus, it may be easier to i.e., regulate daily intake of nicotine.

According to an advantageous embodiment of the invention, the tablet comprises at least one sugar alcohol.

An advantage of the above embodiment may be that a desirable sweetness is provided, e.g. for support of the flavor profile, while inducing saliva segregation as a facilitator for tablet disintegration and nicotine release.

In an embodiment of the invention, the sugar alcohol is a solid sugar alcohol at 25 degrees Celsius.

The solid sugar alcohols have a desirable compressibility; hence the use of solid sugar alcohols is desirable when forming compressed tablets.

It is noted that sugar alcohols, also sometimes referred to as polyols, are different from sugars.

According to an embodiment of the invention, the sugar alcohol is selected from sugar alcohols comprising at least 4 carbon atoms, such as 4 to 16 carbon atoms, such as 5 to 12 carbon atoms.

In an embodiment of the invention, the tablet is free of sugar alcohols comprising no more than three carbons. Examples of sugar alcohols comprising no more than three carbons include glycerol, propylene glycol, and ethylene glycol.

In an embodiment, the nicotine tablet is free of glycerol.

According to an advantageous embodiment of the invention, the tablet comprises the at least one sugar alcohol sugar alcohol in an amount of at least 40% by weight of the tablet, such as at least 50% by weight of the tablet, such as at least 60% by weight of the tablet, such as at least 70% by weight of the tablet, such as at least 80% by weight of the tablet.

In an embodiment of the invention, the tablet comprises the at least one sugar alcohol in an amount of 40 to 95% by weight of the tablet, such as 50 to 95% by weight of the tablet, such as 60 to 90% by weight of the tablet, such as 70 to 90% by weight of the tablet, such as 80 to 90% by weight of the tablet.

According to an advantageous embodiment of the invention, the at least one sugar alcohol comprises sugar alcohol selected from the group consisting of xylitol, maltitol, mannitol, erythritol, isomalt, sorbitol, lactitol and any combination thereof.

Thus, in the above embodiment, the at least one sugar alcohol may include mixtures of different types of sugar alcohols.

In an embodiment of the invention, the at least one sugar alcohol is selected from the group consisting of xylitol, maltitol, mannitol, erythritol, isomalt, sorbitol, lactitol and any combination thereof.

In an embodiment of the invention, the at least one sugar alcohol is selected from the group consisting of xylitol, maltitol, mannitol, isomalt, sorbitol, and any combination thereof.

In an embodiment of the invention, the at least one sugar alcohol is selected from the group consisting of xylitol, mannitol, isomalt, sorbitol and any combination thereof.

In an embodiment of the invention, the at least one sugar alcohol is selected from the group consisting of mannitol, isomalt, sorbitol and any combination thereof.

In an embodiment of the invention, the at least one sugar alcohol comprises or consists of mannitol.

In an embodiment of the invention, the at least one sugar alcohol comprises or consists of isomalt.

In an embodiment of the invention, the at least one sugar alcohol comprises or consists of sorbitol.

In an embodiment of the invention, the tablet comprises at least one sugar alcohol is selected from the group consisting of xylitol, maltitol, mannitol, erythritol, isomalt, sorbitol, lactitol and any combination thereof in an amount of at least 40% by weight of the tablet, such as at least 50% by weight of the tablet, such as at least 60% by weight of the tablet, such as at least 70% by weight of the tablet, such as at least 80% by weight of the tablet.

According to the above embodiment, the tablet may comprise further sugar alcohols not selected from said group consisting of xylitol, maltitol, mannitol, erythritol, isomalt, sorbitol, lactitol and any combination thereof.

According to an advantageous embodiment of the invention, the tablet further comprises a disintegrant.

According to an advantageous embodiment of the invention, the tablet comprises disintegrant in an amount of at least 0.5% by weight of the tablet, such as at least 1% by weight of the tablet, such as at least 2% by weight of the tablet, such as at least 3% by weight of the tablet, such as at least 5% by weight of the tablet.

According to an embodiment of the invention, the tablet comprises disintegrant in an amount of 0.5 to 15% by weight of the tablet, such as 1 to 15% by weight of the tablet, such as 2 to 14% by weight of the tablet, such as 3 to 12% by weight of the tablet, such as 5 to 10% by weight of the tablet.

According to an advantageous embodiment of the invention, the disintegrant is selected from the list consisting of starch, pregelatinated starch, modified cellulose, alginates, ion-exchange resin, calcium silicate, crosslinked cellulose, crosslinked polyvinyl pyrrolidone, crosslinked starch, crosslinked alginic acid, and combinations thereof.

According to an embodiment of the invention, the disintegrant is selected from the list consisting of starch, pregelatinated starch, cellulose, modified cellulose, microcrystalline cellulose, alginates, ion-exchange resin, calcium silicate, crosslinked cellulose, crosslinked polyvinyl pyrrolidone, crosslinked starch, crosslinked alginic acid, and combinations thereof.

In the present context starch refer to starches of various origin such as potato starch, corn starch, wheat starch, pea starch etc.

Examples of pregelatinized starch disintegrants include structure and brands names such as pregelatinized potato starch, pregelatinized wheat starch, pregelatinized corn starch, Lycatab^{®}, starch 1500^{®} etc.

Examples of modified cellulose disintegrants include structure names such as methyl cellulose, carboxymethyl cellulose, hydroxypropyl cellulose, hydroxypropyl methylcellulose etc.

Examples of alginic acid disintegrants include various alkali alginates and brands names such as Vivapur^{®} alginate etc.

It is understood that some disintegrants are referred to as superdisintegrants.

According to an advantageous embodiment of the invention, the disintegrant comprises a superdisintegrant.

According to an advantageous embodiment of the invention, the tablet comprises superdisintegrant in an amount of at least 0.5% by weight of the tablet, such as at least 1% by weight of the tablet, such as at least 2% by weight of the tablet, such as at least 3% by weight of the tablet, such as at least 5% by weight of the tablet.

According to an embodiment of the invention, the tablet comprises superdisintegrant in an amount of 0.5 to 15% by weight of the tablet, such as 1 to 15% by weight of the tablet, such as 2 to 14% by weight of the tablet, such as 3 to 12% by weight of the tablet, such as 5 to 10% by weight of the tablet.

In some embodiments, the disintegrant may comprise combination of regular disintegrant and superdisintegrant.

In an embodiment of the invention, the disintegrant consists of superdisintegrant.

According to an advantageous embodiment of the invention, the superdisintegrant is selected from the group consisting of crosslinked cellulose, crosslinked polyvinyl pyrrolidone, crosslinked starch, crosslinked alginic acid, and any combinations thereof.

Examples of crosslinked cellulose super disintegrants include structure and brands names such as cross-linked sodium carboxymethylcellulose, Croscarmellose^{®}, Ac-Di-Sol^{®}, Solutab^{®} etc.

Examples of crosslinked polyvinyl pyrrolidone (PVP) super disintegrants include structure and brands names such as Kollidon^{®}, Polyplasdone^{®}, polyplasdone XL^{®}, Kollidon CL^{®} etc. Crosslinked polyvinyl pyrrolidone (PVP) disintegrant is also sometimes referred to as crospovidone.

Examples of crosslinked starch super disintegrants include structure and brands names such as sodium starch glycolate, Glycolys^{®}, Explotab^{®}, Primogel^{®}, Vivastart, Tablo^{®} etc.

Examples of cross linked alginic acid include structure and brands names such as Alginic acid NF^{®}, Satialgine^{®} etc.

In an advantageous embodiment of the invention, the disintegrant is a super disintegrant selected from the group consisting of crosslinked cellulose, crosslinked polyvinyl pyrrolidone (PVP), crosslinked starch, and any combinations thereof.

In an advantageous embodiment of the invention, the disintegrant is a super disintegrant selected from the group consisting of crosslinked polyvinyl pyrrolidone, croscarmellose, sodium starch glycolate, and any combinations thereof.

In an embodiment of the invention, the disintegrant comprises cross-linked polyvinylpyrrolidone.

In an embodiment of the invention, at least 50% by weight of the cross-linked polyvinylpyrrolidone has a particle size below 50 micrometers.

In an embodiment of the invention, at least 25% by weight of the cross-linked polyvinylpyrrolidone has a particle size below 15 micrometers.

According to an advantageous embodiment of the invention, the tablet is compressed using a compression force of 1 to 35 kN, such as 2 to 30 kN, such as 2 - 20 kN.

According to an advantageous embodiment of the invention, the nicotine is selected from the group consisting of a nicotine salt, nicotine free base, a nicotine-ion exchange resin combination, a nicotine inclusion complex or nicotine in any non-covalent binding, nicotine bound to zeolites, nicotine bound to cellulose, nicotine bound to starch microspheres, and any combination thereof.

In an embodiment of the invention, the nicotine is selected from the list consisting of nicotine free base and nicotine salts, or combinations thereof.

According to an advantageous embodiment of the invention, the nicotine comprises nicotine free base.

Free base nicotine includes nicotine mixed with sugar alcohols, modified calcium carbonate, water-soluble fibers, water-insoluble fibers, and combinations thereof.

In an embodiment of the invention, the nicotine is nicotine free base.

In an embodiment of the invention, the nicotine comprises a nicotine inclusion complex. In an embodiment of the invention, the nicotine comprises nicotine bound to an ion exchange resin. In an embodiment of the invention, the nicotine comprises nicotine bound to polacrilex resin. As used herein, the term "NPR" refers to nicotine bound to polacrilex resin.

According to an advantageous embodiment of the invention, the nicotine comprises nicotine salt.

An advantage of the above embodiment may be that a fast craving relief of nicotine may be facilitated, e.g. due to a fast dissociation of the nicotine salt.

In an embodiment of the invention, the nicotine is a nicotine salt.

According to an advantageous embodiment of the invention, the nicotine salt is selected from the group consisting of nicotine ascorbate, nicotine aspartate, nicotine benzoate, nicotine monotartrate, nicotine bitartrate, nicotine chloride, nicotine citrate, nicotine fumarate, nicotine lactate, nicotine mucate, nicotine laurate, nicotine levulinate, nicotine malate, nicotine perchlorate, nicotine pyruvate, nicotine salicylate, nicotine sorbate, nicotine succinate, nicotine sulfate, and any combination thereof.

It is understood that the nicotine salt may also be provided as a hydrated salt, or as a combination of hydrated and non-hydrated salt.

Nicotine chloride may e.g. be nicotine hydrochloride and/or nicotine dihydrochloride.

In an embodiment of the invention, the nicotine salt is selected from the list consisting of nicotine benzoate, nicotine monotartrate, nicotine bitartrate, nicotine hydrochloride, nicotine dihydrochloride, nicotine lactate, nicotine malate, nicotine pyruvate, nicotine succinate, and combinations thereof.

According to an advantageous embodiment of the invention, the nicotine comprises nicotine bitartrate.

The term NBT may be used as an abbreviation to denote nicotine bitartrate.

An advantage of the above embodiment may be that a fast craving relief of nicotine may be facilitated, e.g. due to a fast dissociation of nicotine bitartrate.

In an embodiment of the invention, the nicotine is nicotine bitartrate.

In an embodiment of the invention, said nicotine is provided in ionic complex with at least one mucoadhesive water-soluble anionic polymer.

In an embodiment of the invention, the nicotine comprises synthetic nicotine.

In an embodiment of the invention, the nicotine consists of synthetic nicotine.

According to an advantageous embodiment of the invention, the tablet comprises nicotine in an amount of at least 0.2 mg, such as at least 0.5 mg, such as at least 1.0 mg.

In an embodiment of the invention, the tablet comprises nicotine in an amount of 0.2 mg to 12.0 mg, such as 0.2 mg to 10.0 mg, such as 0.5 mg to 8.0 mg, such as 0.5 mg to 6.0 mg, such as 1.0 mg to 4.0 mg, such as 1.0 to 3.0 mg, such as 1.0 to 2.0 mg.

According to an advantageous embodiment of the invention, the tablet comprises nicotine in an amount of at least 0.2% by weight of the tablet, such as at least 0.3% by weight of the tablet, such as at least 0.5% by weight of the tablet.

In an embodiment of the invention, the tablet comprises nicotine in an amount of 0.2 to 5% by weight of the tablet, such as 0.3 to 3% by weight of the tablet, such as 0.5 to 2% by weight of the tablet.

According to an advantageous embodiment of the invention, the pH regulating agent is an alkaline pH regulating agent, such as an alkaline buffering agent.

In an embodiment of the invention, the pH regulating agent is free of acidic pH regulating agent.

In an embodiment of the invention, the pH regulating agent is a buffering agent.

According to an advantageous embodiment of the invention, the pH regulating agent comprises pH regulating agent selected from the group consisting of sodium carbonate, sodium bicarbonate, potassium carbonate, potassium bicarbonate, trometamol, amino acids, di-alkali hydrogen phosphate, tri-alkali phosphate, or any combination thereof.

In an embodiment of the invention, the pH regulating agent is selected from the group consisting of sodium carbonate, sodium bicarbonate, potassium carbonate, potassium bicarbonate, trometamol, amino acids, disodium hydrogen phosphate, dipotassium hydrogen phosphate, trisodium phosphate, tripotassium phosphate, or any combination thereof.

In an embodiment of the invention, the pH regulating agent is selected from the group consisting of sodium carbonate, sodium bicarbonate, potassium carbonate, potassium bicarbonate, trometamol, amino acids, or any combination thereof.

According to an advantageous embodiment of the invention, the pH regulating agent is selected from the group consisting of sodium carbonate, sodium bicarbonate, potassium carbonate, potassium bicarbonate, or any combination thereof.

Combinations of a carbonate and a bicarbonate may be especially advantageous. Such combination may e.g. be a sodium carbonate - sodium bicarbonate buffer system, e.g. sodium carbonate and sodium bicarbonate in a weight-ratio between 5:1 and 1:1, preferably in a weight-ratio between 4.1:1 and 1.5:1.

According to an advantageous embodiment of the invention, the pH regulating agent comprises or consists of sodium carbonate.

An advantage of the above embodiment may be an increased uptake of nicotine in the oral cavity.

In an embodiment of the invention, the pH regulating agent is selected from the group consisting of trometamol, amino acids, disodium hydrogen phosphate, dipotassium hydrogen phosphate, trisodium phosphate, tripotassium phosphate, or any combination thereof.

In an embodiment of the invention, the pH regulating agent is selected from the group consisting of trometamol, disodium hydrogen phosphate, dipotassium hydrogen phosphate, trisodium phosphate, tripotassium phosphate, or any combination thereof.

In an embodiment of the invention, the pH regulating agent comprises di-alkali hydrogen phosphate and/or tri-alkali phosphate, such as disodium phosphate, dipotassium phosphate, trisodium phosphate and/or tripotassium phosphate.

Trometamol and phosphate buffers have a desirable relative neutral taste, hence the use of these pH regulating agents may be found not compromise the taste and mouthfeel of the nicotine tablet.

In an embodiment of the invention, the pH regulating agent comprises trometamol.

In an embodiment of the invention, the pH regulating agent consists of trometamol.

In the present context the term trometamol refers to (tris(hydroxymethyl)aminomethane), also sometimes referred to as tris buffer.

In an embodiment of the invention, the pH regulating agent comprises amino acid.

In an embodiment of the invention, the pH regulating agent consists of amino acid.

According to an advantageous embodiment of the invention, the tablet comprises the pH regulating agent in an amount of at least 0.2% by weight of the tablet, such as at least 0.5% by weight of the tablet, such as at least 1% by weight of the tablet, such as at least 2% by weight of the tablet.

In an embodiment of the invention, the tablet comprises the pH regulating agent in an amount of 0.2 to 10% by weight of the tablet, such as 0.5 to 8% by weight of the tablet, such as 1 to 6% by weight of the tablet, such as 2 to 4% by weight of the tablet.

According to an advantageous embodiment of the invention, the tablet has a weight of no more than 600 mg, such as no more than 500 mg, such as no more than 400 mg, such as no more than 300 mg, such as no more than 200 mg, such as no more than 150 mg, such as no more than 120 mg, such as no more than 100 mg.

In an embodiment of the invention, the tablet has a weight of 25 to 600 mg, such as 25 to 500 mg, such as 25 to 400 mg, such as 25 to 300 mg, such as 25 to 200mg, such as 50 to 150 mg, such as 70-120mg, such as about 75 mg or about 100 mg.

An advantage of the above embodiment may be that the size may facilitate a discreet placement under the lip.

According to an advantageous embodiment of the invention, the tablet is a multilayer tablet comprising a first layer and a second layer.

According to an embodiment of the invention, the first and the second layers are fused by compression.

According to an advantageous embodiment of the invention, the tablet is a one-layer tablet.

In the present context the term layer refers to a module of the tablet composing a significant part of the tablet, such as e.g. at least 20% by weight of the tablet, such as at least 30% by weight of the tablet, such as at least 40% by weight of the tablet.

In an embodiment of the invention, the tablet comprises microcrystalline cellulose, e.g. in an amount of 1-10 % by weight of the formulation.

Microcrystalline cellulose is a refined form of natural cellulose. Examples of microcrystalline cellulose disintegrants include brands names such as Avicel^{®}, Emcocel^{®}, Vivapur^{®} MCC etc.

In an embodiment of the invention, the composition further comprises an amount of an insoluble composition.

In an embodiment of the invention, the insoluble composition comprises at least one selected from dibasic calcium phosphate, calcium carbonate, hydrogenated vegetable oil, and combinations thereof.

In an embodiment of the invention, the amount of the insoluble composition is between 5 and 50 % by weight of the formulation, such as between 10 and 30% by weight of the formulation.

According to an advantageous embodiment of the invention, the tablet is free of effervescent agents.

An advantage of the above embodiment may be an improved user experience. Often, inclusion of effervescent agents may lead to tingling sensations, which may be rather undesirable when experienced between the gum and the lip.

A further advantage may be that using effervescent agents may lead to the oral pH value being below the optimum range for efficient nicotine absorption over the mucous membrane. Avoiding the use of effervescent agents may therefore facilitate improved nicotine absorption.

According to an advantageous embodiment of the invention, the tablet further comprises dissolution modifiers in an amount of at most 1% by weight of the tablet, such as at most 0.5% by weight of the tablet, such at most 0.25% by weight of the tablet, such at most 0.1% by weight of the tablet.

In an embodiment of the invention, the tablet comprises small amount of dissolution modifiers selected from the group consisting of xanthan gum, guar gum, and any combinations thereof.

An advantage of the above embodiment may be that the small amount of dissolution modifiers may improve the mouthfeel.

In an embodiment of the invention, the dissolution modifier comprises xanthan gum.

In an embodiment of the invention, the dissolution modifier comprises guar gum.

According to an advantageous embodiment of the invention, the tablet is free of dissolution modifiers.

In an embodiment of the invention, the tablet is substantially free of dissolution modifiers, such as free of dissolution modifiers.

In an embodiment of the invention, the tablet is free of xanthan gum.

According to an advantageous embodiment of the invention, the tablet is free of mucoadhesives.

Thus, according to an embodiment of the invention, the tablet is not an adhering tablet, e.g. by not including any mucoadhesive, or by only including a minor amount of mucoadhesive, such as no more than 10% by weight of the tablet, such as no more than 5% by weight of the tablet, such as no more than 2% by weight of the tablet, such as no more than 1% by weight of the tablet.

According to an advantageous embodiment of the invention, the tablet comprises flavor.

According to an embodiment of the invention, the tablet comprises liquid flavor.

According to an embodiment of the invention, the tablet comprises powdered flavor.

According to an embodiment of the invention, the tablet comprises powdered flavor and liquid flavor.

An advantage of the above embodiment may be that flavor improve the mouthfeel and reduce sense of burning.

According to an advantageous embodiment of the invention, the tablet is free of flavor.

According to an embodiment of the invention, the tablet is free of liquid flavor.

According to an embodiment of the invention, the tablet is free of powdered flavor.

In an embodiment of the invention, the tablet comprises a lubricant, such as sodium stearyl fumarate (SSF) or magnesium stearate.

According to an advantageous embodiment of the invention, the maximum plasma concentration (Cmax) of nicotine is increased by administration between the lip and gum compared to administration on the tongue.

According to an advantageous embodiment of the invention, the maximum plasma concentration (Cmax) of nicotine is increased by at least 10% by administration between the lip and gum compared to administration on the tongue, such as by at least 20% by administration between the lip and gum compared to administration on the tongue, such as by at least 30% by administration between the lip and gum compared to administration on the tongue.

According to an advantageous embodiment of the invention, the maximum plasma concentration (Cmax) of nicotine is increased by administration between the lip and gum compared to sublingual administration.

According to an advantageous embodiment of the invention, the maximum plasma concentration (Cmax) of nicotine is increased by at least 10% by administration between the lip and gum compared to sublingual administration, such as by at least 20% by administration between the lip and gum compared to sublingual administration, such as by at least 30% by administration between the lip and gum compared to sublingual administration.

According to an advantageous embodiment of the invention, the area under the curve (AUC) for nicotine is increased by administration between the lip and gum compared to administration on the tongue.

According to an advantageous embodiment of the invention, the area under the curve (AUC) for nicotine is increased by at least 10% by administration between the lip and gum compared to administration on the tongue, such as by at least 15% by administration between the lip and gum compared to administration on the tongue, such as by at least 20% by administration between the lip and gum compared to administration on the tongue.

According to an advantageous embodiment of the invention, the area under the curve (AUC) for nicotine is increased by administration between the lip and gum compared to sublingual administration.

According to an advantageous embodiment of the invention, the area under the curve (AUC) for nicotine is increased by at least 10% by administration between the lip and gum compared to sublingual administration, such as by at least 15% by administration between the lip and gum compared to sublingual administration, such as by at least 20% by administration between the lip and gum compared to sublingual administration.

According to an advantageous embodiment of the invention, the time (Tmax) until maximum plasma concentration (Cmax) of nicotine occurs is decreased by administration between the lip and gum compared to administration on the tongue.

According to an advantageous embodiment of the invention, the time (Tmax) until maximum plasma concentration (Cmax) of nicotine occurs is decreased by at least 10% by administration between the lip and gum compared to administration on the tongue, such as by at least 20% by administration between the lip and gum compared to administration on the tongue, such as by at least 30% by administration between the lip and gum compared to administration on the tongue.

According to an advantageous embodiment of the invention, the tablet is free of nicotine bound to a ceramic material.

According to an advantageous embodiment of the invention, the tablet is free of ceramic material.

The invention further relates to an orally disintegrating nicotine composition for use in alleviation of nicotine craving, the composition comprising nicotine and a pH regulating agent, wherein the composition is administered between the lip and gum.

According to an advantageous embodiment of the invention, orally disintegrating nicotine composition according to the invention or any of its embodiments is composed as the orally disintegrating nicotine tablet according to the invention or any of its embodiments.

In particular, it is noted how the composition may in advantageous embodiments comprise disintegrants as described above in relation to the orally disintegrating nicotine tablet of the invention and any of its embodiments.

The invention further relates to a method for treatment of nicotine craving, the method comprising the steps of administering a therapeutically effective amount of nicotine, wherein the nicotine is provided in an orally disintegrating nicotine tablet comprising the nicotine and a pH regulating agent, and wherein the tablet is positioned between the lip and gum.

Thus, the orally disintegrating nicotine tablet may advantageously be provided to a subject in need therefore, in particular for relief of nicotine craving.

It is noted that the tablet is designed to disintegrate within a period of 300 seconds, such as within 270 seconds, such as within 240 seconds, and thus in an embodiment of the invention, the method further comprises a step of disintegrating the tablet within a period of 300 seconds, such as 270 seconds, such as 240 seconds. It is noted that the tablet is completely disintegrated after 300 seconds, such as 270 seconds, such as 240 seconds.

According to an advantageous embodiment of the invention, the tablet is positioned between the upper lip and gum.

According to an advantageous embodiment of the invention, the nicotine is provided as the orally disintegrating nicotine tablet according to the invention or any of its embodiments.

The invention further relates to aspects and embodiments according to the following clauses.

Clause 1. An orally disintegrating nicotine tablet for use in alleviation of nicotine craving,
the tablet comprising nicotine and a pH regulating agent, wherein the tablet is administered between the lip and gum.

Clause 2. The orally disintegrating nicotine tablet according to clause 1, wherein the tablet is administered between the upper lip and gum.

Clause 3. The orally disintegrating nicotine tablet according to clause 1 or 2, wherein the tablet is designed to disintegrate within a period of 300 seconds upon oral administration between the lip and gum, such as within 270 seconds upon oral administration between the lip and gum, such as within 240 seconds upon oral administration, such as within a period of 210 seconds upon oral administration, such as within a period of 180 seconds upon oral administration, such as within a period of 150 seconds upon oral administration, such as within a period of 120 seconds upon oral administration, such as within a period of 90 seconds upon oral administration, such as within a period of 60 seconds upon oral administration.

Clause 4. The orally disintegrating nicotine tablet according to any of clauses 1-3, wherein the tablet is designed to disintegrate within a period of 60 seconds in vitro, such as within 50 seconds in vitro, such as within 40 seconds in vitro.

Clause 5. The orally disintegrating nicotine tablet according to any of clauses 1-4, wherein the tablet is a compressed orally disintegrating nicotine tablet.

Clause 6. The orally disintegrating nicotine tablet according to any of clauses 1-5, wherein the tablet is composed of a plurality of compressed particles.

Clause 7. The orally disintegrating nicotine tablet according to any of clauses 1-6, wherein the tablet comprises at least one sugar alcohol.

Clause 8. The orally disintegrating nicotine tablet according to any of clauses 1-7, wherein the tablet comprises the at least one sugar alcohol sugar alcohol in an amount of at least 40% by weight of the tablet, such as at least 50% by weight of the tablet, such as at least 60% by weight of the tablet, such as at least 70% by weight of the tablet, such as at least 80% by weight of the tablet.

Clause 9. The orally disintegrating nicotine tablet according to any of clauses 1-8, wherein the at least one sugar alcohol comprises sugar alcohol selected from the group consisting of xylitol, maltitol, mannitol, erythritol, isomalt, sorbitol, lactitol and any combination thereof.

Clause 10. The orally disintegrating nicotine tablet according to any of clauses 1-9, wherein the tablet further comprises a disintegrant.

Clause 11. The orally disintegrating nicotine tablet according to any of clauses 1-10, wherein the tablet comprises disintegrant in an amount of at least 0.5% by weight of the tablet, such as at least 1% by weight of the tablet, such as at least 2% by weight of the tablet, such as at least 3% by weight of the tablet, such as at least 5% by weight of the tablet.

Clause 12. The orally disintegrating nicotine tablet according to any of clauses 1-11, wherein the disintegrant is selected from the list consisting of starch, pregelatinated starch, modified cellulose, alginates, ion-exchange resin, calcium silicate, crosslinked cellulose, crosslinked polyvinyl pyrrolidone, crosslinked starch, crosslinked alginic acid, and combinations thereof.

Clause 13. The orally disintegrating nicotine tablet according to any of clauses 1-12, wherein the disintegrant comprises a superdisintegrant.

Clause 14. The orally disintegrating nicotine tablet according to any of clauses 1-12, wherein the tablet comprises superdisintegrant in an amount of at least 0.5% by weight of the tablet, such as at least 1% by weight of the tablet, such as at least 2% by weight of the tablet, such as at least 3% by weight of the tablet, such as at least 5% by weight of the tablet.

Clause 15. The orally disintegrating nicotine tablet according to any of clauses 13-14, wherein the superdisintegrant is selected from the group consisting of crosslinked cellulose, crosslinked polyvinyl pyrrolidone, crosslinked starch, crosslinked alginic acid, and any combinations thereof.

Clause 16. The orally disintegrating nicotine tablet according to any of clauses 13-15, wherein the disintegrant is a super disintegrant selected from the group consisting of crosslinked cellulose, crosslinked polyvinyl pyrrolidone (PVP), crosslinked starch, and any combinations thereof.

Clause 17. The orally disintegrating nicotine tablet according to any of clauses 13-16, wherein the disintegrant is a super disintegrant selected from the group consisting of crosslinked polyvinyl pyrrolidone, croscarmellose, sodium starch glycolate, and any combinations thereof.

Clause 18. The orally disintegrating nicotine tablet according to any of clauses 1-17, wherein the tablet is compressed using a compression force of 1 to 35 kN, such as 2 to 30 kN, such as 2 - 20 kN.

Clause 19. The orally disintegrating nicotine tablet according to any of clauses 1-18, wherein the nicotine is selected from the group consisting of a nicotine salt, nicotine free base, a nicotine-ion exchange resin combination, a nicotine inclusion complex or nicotine in any non-covalent binding, nicotine bound to zeolites, nicotine bound to cellulose, nicotine bound to starch microspheres, and any combination thereof.

Clause 20. The orally disintegrating nicotine tablet according to any of clauses 1-19, wherein the nicotine comprises nicotine free base.

Clause 21. The orally disintegrating nicotine tablet according to any of clauses 1-20, wherein the nicotine comprises nicotine salt.

Clause 22. The orally disintegrating nicotine tablet according to clause 21, wherein the nicotine salt is selected from the group consisting of nicotine ascorbate, nicotine aspartate, nicotine benzoate, nicotine monotartrate, nicotine bitartrate, nicotine chloride, nicotine citrate, nicotine fumarate, nicotine lactate, nicotine mucate, nicotine laurate, nicotine levulinate, nicotine malate, nicotine perchlorate, nicotine pyruvate, nicotine salicylate, nicotine sorbate, nicotine succinate, nicotine sulfate, and any combination thereof.

Clause 23. The orally disintegrating nicotine tablet according to any of clauses 1-22, wherein the nicotine comprises nicotine bitartrate.

Clause 24. The orally disintegrating nicotine tablet according to any of clauses 1-23, wherein the tablet comprises nicotine in an amount of at least 0.2 mg, such as at least 0.5 mg, such as at least 1.0 mg.

Clause 25. The orally disintegrating nicotine tablet according to any of clauses 1-24, wherein the tablet comprises nicotine in an amount of at least 0.2% by weight of the tablet, such as at least 0.3% by weight of the tablet, such as at least 0.5% by weight of the tablet.

Clause 26. The orally disintegrating nicotine tablet according to any of clauses 1-25, wherein the pH regulating agent is an alkaline pH regulating agent, such as an alkaline buffering agent.

Clause 27. The orally disintegrating nicotine tablet according to any of clauses 1-26, wherein the pH regulating agent comprises pH regulating agent selected from the group consisting of sodium carbonate, sodium bicarbonate, potassium carbonate, potassium bicarbonate, trometamol, amino acids, di-alkali hydrogen phosphate, tri-alkali phosphate, or any combination thereof.

Clause 28. The orally disintegrating nicotine tablet according to any of clauses 1-27, wherein the pH regulating agent is selected from the group consisting of sodium carbonate, sodium bicarbonate, potassium carbonate, potassium bicarbonate, or any combination thereof.

Clause 29. The orally disintegrating nicotine tablet according to any of clauses 1-28, wherein the pH regulating agent comprises or consists of sodium carbonate.

Clause 30. The orally disintegrating nicotine tablet according to any of clauses 1-29, wherein the tablet comprises the pH regulating agent in an amount of at least 0.2% by weight of the tablet, such as at least 0.5% by weight of the tablet, such as at least 1% by weight of the tablet, such as at least 2% by weight of the tablet.

Clause 31. The orally disintegrating nicotine tablet according to any of clauses 1-30, wherein the tablet has a weight of no more than 600 mg, such as no more than 500 mg, such as no more than 400 mg, such as no more than 300 mg, such as no more than 200 mg, such as no more than 150 mg, such as no more than 120 mg, such as no more than 100 mg.

Clause 32. The orally disintegrating nicotine tablet according to any of clauses 1-31, wherein the tablet is a multilayer tablet comprising a first layer and a second layer.

Clause 33. The orally disintegrating nicotine tablet according to any of clauses 1-32, wherein the tablet is a one-layer tablet.

Clause 34. The orally disintegrating nicotine tablet according to any of clauses 1-33, wherein the tablet is free of effervescent agents.

Clause 35. The orally disintegrating nicotine tablet according to any of clauses 1-34, wherein the tablet further comprises dissolution modifiers in an amount of at most 1% by weight of the tablet, such as at most 0.5% by weight of the tablet, such at most 0.25% by weight of the tablet, such at most 0.1% by weight of the tablet.

Clause 36. The orally disintegrating nicotine tablet according to any of clauses 1-35, wherein the tablet is free of dissolution modifiers.

Clause 37. The orally disintegrating nicotine tablet according to any of clauses 1-36, wherein the tablet is free of mucoadhesives.

Clause 38. The orally disintegrating nicotine tablet according to any of clauses 1-37, wherein the tablet comprises flavor.

Clause 39. The orally disintegrating nicotine tablet according to any of clauses 1-38, wherein the tablet is free of flavor.

Clause 40. The orally disintegrating nicotine tablet according to any of clauses 1-39, wherein the maximum plasma concentration (Cmax) of nicotine is increased by administration between the lip and gum compared to administration on the tongue.

Clause 41. The orally disintegrating nicotine tablet according to any of clauses 1-40, wherein the maximum plasma concentration (Cmax) of nicotine is increased by at least 10% by administration between the lip and gum compared to administration on the tongue, such as by at least 20% by administration between the lip and gum compared to administration on the tongue, such as by at least 30% by administration between the lip and gum compared to administration on the tongue.

Clause 42. The orally disintegrating nicotine tablet according to any of clauses 1-41, wherein the maximum plasma concentration (Cmax) of nicotine is increased by administration between the lip and gum compared to sublingual administration.

Clause 43. The orally disintegrating nicotine tablet according to any of clauses 1-42, wherein the maximum plasma concentration (Cmax) of nicotine is increased by at least 10% by administration between the lip and gum compared to sublingual administration, such as by at least 20% by administration between the lip and gum compared to sublingual administration, such as by at least 30% by administration between the lip and gum compared to sublingual administration.

Clause 44. The orally disintegrating nicotine tablet according to any of clauses 1-43, wherein the area under the curve (AUC) for nicotine is increased by administration between the lip and gum compared to administration on the tongue.

Clause 45. The orally disintegrating nicotine tablet according to any of clauses 1-44, wherein the area under the curve (AUC) for nicotine is increased by at least 10% by administration between the lip and gum compared to administration on the tongue, such as by at least 15% by administration between the lip and gum compared to administration on the tongue, such as by at least 20% by administration between the lip and gum compared to administration on the tongue.

Clause 46. The orally disintegrating nicotine tablet according to any of clauses 1-45, wherein the area under the curve (AUC) for nicotine is increased by administration between the lip and gum compared to sublingual administration.

Clause 47. The orally disintegrating nicotine tablet according to any of clauses 1-46, wherein the area under the curve (AUC) for nicotine is increased by at least 10% by administration between the lip and gum compared to sublingual administration, such as by at least 15% by administration between the lip and gum compared to sublingual administration, such as by at least 20% by administration between the lip and gum compared to sublingual administration.

Clause 48. The orally disintegrating nicotine tablet according to any of clauses 1-47, wherein the time (Tmax) until maximum plasma concentration (Cmax) of nicotine occurs is decreased by administration between the lip and gum compared to administration on the tongue.

Clause 49. The orally disintegrating nicotine tablet according to any of clauses 1-48, wherein the time (Tmax) until maximum plasma concentration (Cmax) of nicotine occurs is decreased by at least 10% by administration between the lip and gum compared to administration on the tongue, such as by at least 20% by administration between the lip and gum compared to administration on the tongue, such as by at least 30% by administration between the lip and gum compared to administration on the tongue.

Clause 50. The orally disintegrating nicotine tablet according to any of clauses 1-49, wherein the tablet is free of nicotine bound to a ceramic material.

Clause 51. The orally disintegrating nicotine tablet according to any of clauses 1-50, wherein the tablet is free of ceramic material.

Clause 52. An orally disintegrating nicotine composition for use in alleviation of nicotine craving,
the composition comprising nicotine and a pH regulating agent, wherein the composition is administered between the lip and gum.

Clause 53. The orally disintegrating nicotine composition according to clause 52 and any of clauses 1-51.

Clause 54. A method for treatment of nicotine craving, the method comprising the steps of
administering a therapeutically effective amount of nicotine, wherein the nicotine is provided in an orally disintegrating nicotine tablet comprising the nicotine and a pH regulating agent, and wherein the tablet is positioned between the lip and gum.

Clause 55. The method according to clause 54, wherein the tablet is positioned between the upper lip and gum.

Clause 56. The method according to clause 54 or 55, wherein the orally disintegrating nicotine tablet is in accordance with any of clauses 1-51.

### FIGURES

The invention will now be described with reference to the figures, where
Figure 1 illustrates perceived effect of an example of an orally disintegrating nicotine tablet used according to the invention,
Figure 2 illustrates effect on pulse rate of an example of an orally disintegrating nicotine tablet used according to the invention,
Figure 3 illustrates nicotine plasma concentration of an example of an orally disintegrating nicotine tablet used according to the invention and a nicotine mouthspray,
Figure 4 illustrates nicotine plasma concentration of an example of an orally disintegrating nicotine tablet used according to the invention and compared with conventional use,
Figure 5 illustrates nicotine plasma concentration of examples of an orally disintegrating nicotine tablets with different nicotine doses used according to the invention, and
Figure 6 illustrates nicotine induced throat irritation known as nicotine burning resulting from an example of an orally disintegrating nicotine tablet used according to the invention and compared with conventional use.

### DETAILED DESCRIPTION

As used herein, the term "orally disintegrating tablet" refers to a tablet for oral administering which disintegrates in the oral cavity relatively fast from the administering. However, according to the present invention, the orally disintegrating nicotine tablet is administered between the lip and the gum, preferably between the upper lip and the gum.

Orally disintegrating tablets may also be referred to "orally dissolving tablets", and these two terms are used interchangeably herein. Commonly, these terms are also referred to by their abbreviation, ODT. Similarly, the terms "fast dissolving tablet" and "fast disintegrating tablet", as well as the abbreviation FDT, refers herein to an orally disintegrating tablet. It is noted that orally disintegrating tablets are considered fully disintegrable, i.e. that they completely disintegrate when administered to the oral cavity. In some embodiments, the orally disintegrating tablets are composed solely of water-soluble substances and are therefore completely water-soluble. In other embodiments, the orally disintegrating tablets comprise some amounts of water-insoluble compositions, as long as these do not prevent the tablet from fully disintegrating.

As used herein, the term "disintegrate" refers to a reduction of a said object to components, fragments or particles. Disintegration may both refer to in vivo disintegration, the methods of which are defined herein, or in vitro disintegration. The in vitro measurements are carried out in accordance to European Pharmacopeia 9.0, section 2.9.1, Disintegration of tablets and capsules.

As used herein, the term "dissolve" is the process where a solid substance enters a solvent (oral saliva) to yield a solution. Unless otherwise stated, dissolving implies a full dissolving of the compound in question.

As used herein, the terms "disintegrant" refers to an ingredient facilitating disintegration of an orally disintegrating tablet, when the orally disintegrating tablet comes into contact with saliva. Disintegrants usable within the scope of the invention may include starch, pregelatinated starch, modified starch (including potato starch, maize starch, starch 1500, sodium starch glycolate and starch derivatives), alginates, ion-exchange resin, and superdisintegrants, such as crosslinked cellulose (such as sodium carboxy methyl cellulose), crosslinked polyvinyl pyrrolidone (PVP), crosslinked starch, crosslinked alginic acid, natural superdisintegrants, and calcium silicate. Disintegrants may often be considered as measure promoting the break-up of the dosage form into smaller fragments upon administration to allow the onset of drug dissolution and eventual absorption.

As used herein, the term "nicotine" refers to nicotine in any form, including free base nicotine, nicotine salts, nicotine bound to ion exchange resins, such as nicotine polacrilex, nicotine bound to zeolites; nicotine bound to cellulose, such as microcrystalline cellulose, such as of microbial origin, or starch microspheres, nicotine bound to CaCO3, and mixtures thereof. Thus, when referring to nicotine amounts, the amounts refers to the amount of pure nicotine. Thus, when measuring the concentration of nicotine added as nicotine salt, it is the mass of the equivalent amount of pure nicotine, not the mass of the salt, that is relevant. Nicotine also covers nicotine not obtained from tobacco, often referred to as synthetic nicotine.

As used herein, the term "nicotine salt" refers to nicotine in ionized form bonded electrostatically to a counterion.

As used herein, the term "NBT" refers to nicotine bitartrate and hydrates thereof.

As used herein, the term "%" and "percent" refers to percent by weight, unless otherwise is stated.

As used herein, the term "pH regulating agent" refers to agents, which active adjust and regulates the pH value of the solution to which they have been added or are to be added. Thus, pH regulating agents may be acids and bases, including acidic buffering agents and alkaline buffering agents. On the other hand, pH regulating agents does not include substances and compositions that can only affect the pH by dilution. Furthermore, pH regulating agents does not include e.g. flavoring, fillers, etc. In an advantageous embodiment of the invention, the pH regulating agent is alkaline.

As used herein, the term "buffering agent" is used interchangeably with "buffer" and refers to agents for obtaining a buffer solution. Buffering agents include acidic buffering agents, i.e. for obtaining a buffer solution with an acidic pH, and alkaline buffering agents, i.e. for obtaining a buffer solution with an alkaline pH.

In an embodiment of the invention, the tablet comprises a filler. In an embodiment of the invention, the filler is selected form the group consisting of microcrystalline cellulose, magnesium- and calcium carbonate, sodium sulphate, ground limestone, silicate compounds such as magnesium- and aluminum silicate, kaolin and clay, aluminum oxide, silicon oxide, talc, titanium oxide, mono-, di- and tri-calcium phosphates, cellulose polymers, such as wood, starch polymers, fibers and combinations thereof.

In an embodiment of the invention, the tablet comprises a bulk sweetener. In an embodiment of the invention, the bulk sweetener comprises or consists of sugar sweetener and/or sugarless sweetener.

The bulk sweeteners may often support the flavor profile of the formulation.

In an embodiment of the invention, the sugar sweetener is selected from saccharide-containing components, such as sucrose, dextrose, maltose, saccharose, lactose, sorbose, dextrin, trehalose, D-tagatose, dried invert sugar, fructose, levulose, galactose, and the like, alone or in combination. These sugar sweeteners may also be included as a humectant.

In an embodiment of the invention, the sugarless sweeteners is selected from sugar alcohols (also sometimes referred to as polyols), such as sorbitol, erythritol, xylitol, maltitol, mannitol, lactitol, and isomalt.

In an embodiment of the invention, the disintegrant is selected from crospovidone, croscarmellose sodium, and sodium starch glycolate. In an embodiment of the invention, the disintegrant is selected from starch, pregelatinated starch, modified starch (including potato starch, maize starch, starch 1500, sodium starch glycolate and starch derivatives), alginates, ion-exchange resin, and superdisintegrants, such as crosslinked cellulose (such as sodium carboxy methyl cellulose), crosslinked polyvinyl pyrrolidone (PVP), crosslinked starch, crosslinked alginic acid, natural superdisintegrants, and calcium silicate, and combinations thereof.

In an embodiment of the invention, the tablet comprises a high intensity sweetener. In an embodiment of the invention, the high intensity sweetener is selected from sucralose, aspartame, salts of acesulfame, such as acesulfame potassium, alitame, saccharin and its salts, cyclamic acid and its salts, glycyrrhizin, dihydrochalcones, thaumatin, monellin, stevioside and the like, alone or in combination.

In an embodiment of the invention, the tablet comprises one or more flavors. In an embodiment of the invention, one or more flavors are selected from peppermint, menthol, almond, almond amaretto, apple, Bavarian cream, black cherry, black sesame seed, blueberry, brown sugar, bubblegum, butterscotch, cappuccino, caramel, caramel cappuccino, cheesecake (graham crust), cinnamon redhots, cotton candy, circus cotton candy, clove, coconut, coffee, clear coffee, double chocolate, energy cow, graham cracker, grape juice, green apple, Hawaiian punch, honey, Jamaican rum, Kentucky bourbon, kiwi, koolada, lemon, lemon lime, tobacco, maple syrup, maraschino cherry, marshmallow, menthol, milk chocolate, mocha, Mountain Dew, peanut butter, pecan, peppermint, raspberry, banana, ripe banana, root beer, RY 4, spearmint, strawberry, sweet cream, sweet tarts, sweetener, toasted almond, tobacco, tobacco blend, vanilla bean ice cream, vanilla cupcake, vanilla swirl, vanillin, waffle, Belgian waffle, watermelon, whipped cream, white chocolate, wintergreen, amaretto, banana cream, black walnut, blackberry, butter, butter rum, cherry, chocolate hazelnut, cinnamon roll, cola, creme de menthe, eggnog, English toffee, guava, lemonade, licorice, maple, mint chocolate chip, orange cream, peach, pina colada, pineapple, plum, pomegranate, pralines and cream, red licorice, salt water taffy, strawberry banana, strawberry kiwi, tropical punch, tutti frutti, vanilla, or any combination thereof.

According to an embodiment of the invention, flavor may be used as taste masking for the nicotine.

In an embodiment of the invention, the pH regulating agent is a buffering agent. In an embodiment of the invention, the buffering agent is sodium carbonate. In an embodiment of the invention, the buffering agents is selected from carbonate, including monocarbonate, bicarbonate and sesquicarbonate, glycerinate, phosphate, glycerophosphate, acetate, glyconate or citrate of an alkali metal, ammonium, tris buffer, amino acids and mixtures thereof. Encapsulated buffer such as Effersoda may also be used.

Buffering agent in the tablet may be used to obtain the desired pH-values in the saliva of a tablet user.

In some embodiments, the buffering agent comprises sodium carbonate and sodium bicarbonate, e.g. in a weight-ratio between 5:1 and 2.5:1, preferably in a weight-ratio between 4.1:1 and 3.5:1.

A high suitable buffering agent according to advantageous embodiments of the present invention is the sodium carbonate - sodium bicarbonate buffer system.

In an embodiment of the invention, silicon dioxide is used as a glidant. Other glidants usable for the formulation may also be used within the scope of the invention.

In an embodiment of the invention, magnesium stearate is used as a lubricant. Other lubricants usable for the formulation may also be used within the scope of the invention.

In an embodiment of the invention, ready to use systems may be used. Typically, such ready-to-use systems may e.g. replace filler, disintegrant, glidant or similar with a single powder mix. Suitable ready-to-use systems for the purpose, but not limited to, include Pearlitol Flash (Roquette), Pharmaburst 500 (SPI Pharma), Ludiflash (BASF), ProSolv (JRS Pharma), ProSolv EasyTab (JRS Pharma), F-Melt (Fuji Chemical), SmartEx50 or SmartEx100 (Shin Etsu / Harke Pharma).

In order to obtain an orally disintegrating tablet, such as an orally disintegrating tablet disintegrating within 300 seconds upon oral administration between the lip and gum, such as within 270 seconds upon oral administration between the lip and gum, such as within 240 seconds from oral administration between the lip and gum, or an orally disintegrating tablet disintegrating within 60 seconds in vitro, a range of parameters can be adjusted.

First, by varying the composition, the disintegration time can be altered. Using ingredients with a high water-solubility may facilitate a lowered disintegration time.

Particularly, including a disintegrant may significantly influence the disintegration time, subject to the total composition. Also, by varying the amount and type of the disintegrant, the disintegration time may be further adjusted. For example, if a tablet having a lower disintegration time is desired, the percentage content of disintegrant may be increased and/or the type of disintegrant may be at least partly exchanged for a more effective disintegrant. In this respect, superdisintegrants are generally considered more effective disintegrants than disintegrants not being superdisintegrants.

Also, decreasing the particle size of the disintegrant tends to lower the disintegration time, likely due to an increased surface area to volume ratio.

Furthermore, the compression force used in compressed tablets correlate significantly with the obtained hardness, such that a high compression force typically increases the hardness of the obtained tablet. By adjusting the hardness of a tablet, the disintegration time may also be influenced, such that a lowered hardness typically gives a shorter disintegration time. Here it has been observed for a number of compositions that by applying the correct compression force a disintegration time below 300 seconds upon oral administration between the lip and gum, such as below 270 seconds upon oral administration between the lip and gum, such as below 240 second upon oral administration and/or 60 seconds in vitro can be achieved, whereas a too high compression force may result in a longer disintegration time above 300 seconds, 270 seconds, or 240 seconds in vivo or above 60 seconds in vitro. In this regard it is noted that the threshold compression force may vary significantly, depending on other parameters, such as overall composition, content and type of disintegrant, etc. When, for example, a certain setup results in a too slow disintegration, a further way of adjusting may be to replace a regular disintegrant with a superdisintegrant, i.e. which facilitates disintegration in a more efficient way.

Increasing the water-solubility may also be facilitated by exchanging ingredients with low water-solubility with ingredients having higher water-solubility. For example, using sugar alcohols as fillers may be very advantageous insofar that the sugar alcohols have a higher water solubility than alternative fillers.

Moreover, using sugar alcohols with a lower compactibility leads to lower disintegration time. Too low compactibility may compromise the mechanical strength of the tablet and lead to undesirably high friability and risk of cracks etc.

Further examples of parameters that may be adjusted in order to obtain an orally disintegrating nicotine tablet include size and shape of the tablet. The larger the tablet, the longer the disintegration time and thus release time of the nicotine and pH regulating agent.

For example, increasing the flatness (e.g. quantified by a diameter to height ratio) for a disc-shaped tablet typically increases disintegration time by increasing the surface-to-volume. As long as the tablet has a satisfactory mechanical strength, flatness may be increased.

Also, modifying the cross-sectional profile from a convex type tablet to a concave shaped tablet lowers the disintegration time. It is noted that this may to some degree lower the mechanical strength of the tablet, however, as long as it is satisfactory, pursuing the concave cross-section may help to increase disintegration and thus lower the disintegration time.

Also, when using binders, e.g. to obtain a higher cohesiveness and mechanical strength of the tablet, the amount of such binders may be decreased as much as possible to obtain a higher disintegration rate and thus a shorter disintegration time.

Furthermore, by adding a salivation agent to the tablet, an increased amount of saliva in the vicinity of the tablet may be facilitated, which again supports the dissolving and disintegration of the tablet to reduce the disintegration time. Thus, sweeteners, in particular sugar alcohols, may be advantageous in this respect.

Further, the type and amount of lubricant, if any, may be adjusted to optimize disintegration time. For example, using Sodium stearyl fumarate (SSF) typically leads to a lower disintegration time compared to when using magnesium stearate MgSt.

Thus, a wide range of parameters may be adjusted when designing an orally disintegrating nicotine tablet designed for fast disintegrating, such as within a period of 60 seconds in vitro or within 300 seconds from administration between the lip and gum, such as within 270 seconds from administration between the lip and gum, such as within240 from administration between the lip and gum.

Typically, the formulation comprises of ingredients selected from the group consisting of bulk sweeteners, fillers, ready to use systems, flavors, dry-binders, disintegrant, hereunder superdisintegrants, tabletting aids, anti-caking agents, emulsifiers, antioxidants, enhancers, absorption enhancers, buffering agents, high intensity sweeteners, colors, glidants, lubricants, or any combination thereof. Absorption enhancers may include e.g. pH regulating agents, such as buffering agents, and mucoadhesive.

In an embodiment of the invention, the tablet core is provided with an outer coating.

In an embodiment of the invention, said outer coating is selected from the group consisting of hard coating, soft coating and edible film-coating or any combination thereof.

According to an embodiment of the invention, at least a part of the nicotine is adhered to dry-binder particles.

According to an embodiment of the invention, an amount of dry-binder is used to adhere nicotine to bulk sweetener.

According to an embodiment of the invention, said orally disintegrating tablet comprises one or more encapsulation delivery systems.

### EXAMPLES

The following non-limiting examples illustrate different variations of the present invention.

### Example 1

### Preparation of orally disintegrating tablets

In the present example six orally disintegrating tablets (ODT) with 1 mg nicotine are prepared with formulations as outlined in table 1. The orally disintegrating tablets are prepared with NBT (nicotine bitartrate dihydrate). Punch used: 7.00 mm, circular, shallow concave, D tooling. Tablet weight: 100.0 mg.

**Table 1 - Orally disintegrating tablet compositions. Amounts are given in mg. ODT=Orally disintegrating tablet.**

| | **ODT(a)** | **ODT(b)** | **ODT(c)** | **ODT(d)** | **ODT(e)** | **ODT(f)** |
|---|---|---|---|---|---|---|
| NBT | 2.85 | 2.85 | 2.85 | 2.85 | 2.85 | 2.85 |
| Microcrystalline cellulose | - | - | - | 40.175 | 40.175 | 40.175 |
| Mannitol | 81.35 | 81.35 | 81.35 | 40.175 | 40.175 | 40.175 |
| Crospovidone | 5.0 | - | - | 5.0 | - | - |
| Croscarmellose Sodium | - | 5.0 | - | - | 5.0 | - |
| Sodium Starch Glycolate | - | - | 5.0 | - | - | 5.0 |
| Flavor | 4.4 | 4.4 | 4.4 | 4.4 | 4.4 | 4.4 |
| Sucralose | 0.4 | 0.4 | 0.4 | 0.4 | 0.4 | 0.4 |
| Sodium carbonate | 5.0 | 5.0 | 5.0 | 5.0 | 5.0 | 5.0 |
| Silicium dioxide | - | - | - | 1.0 | 1.0 | 1.0 |
| Magnesium stearate | 1.0 | 1.0 | 1.0 | 1.0 | 1.0 | 1.0 |
| Total | 100.0 | 100.0 | 100.0 | 100.0 | 100.0 | 100.0 |

In the above examples a peppermint flavor in powder form was used. A mixture of peppermint and menthol may also be used. Of course, other flavors as described herein may be used as well, in combination with menthol and/or peppermint or replacing these. The flavor may be liquid or powdered or a combination, i.e. a liquid flavor and a powdered flavor is added.

Raw materials are weighed from bags or buckets into separate weighing containers.

All excipients are sifted through an 800 micrometer sieve into a stainless steel or plastic bin in the following order:
- Half the filler / bulk sweetener
- The API and all other excipients, except magnesium stearate
- The remaining half of the filler / bulk sweetener

These are mixed in a Turbula mixer for 4 - 10 minutes at 25 RPM. Then lubricant, for example magnesium stearate is sifted through an 800 micrometer sieve into the mixing bin, and the lubrication is conducted by additional mixing for 1 - 2 minutes at 25 RPM. The fill level of the mixing bin is kept between 40% and 70%, according to standardized practice. The lubricated powder blend is transferred to the hopper of a tableting machine.

The orally disintegrating tablets are manufactured on a lab scale machine, for example RIVA Piccola bi-layer tablet press. The tablet machine is commissioned by adjusting the fill depth and compression force so the weight and hardness of lozenges match the acceptance criteria. A pre-compression force could be included to avoid capping.

**Table 2: Suggested start up parameters. *The design of punches is not fixed. As the curvature impacts thickness, the thickness is not a fixed target at this time of development.**

| **Parameter** | **Target value** |
|---|---|
| Speed | 10 - 20 rpm |
| Weight of ODT | 100 mg +/- 5 % |
| Compression force | 2-8kN |
| Thickness | N/A* |
| Friability (100rpm) | <1% |

The acceptance criteria for friability should be fulfilled so packaging of the resulting orally disintegrating tablets is possible, but in this embodiment, the bulk sweetener and or filler should have relatively good compressibility and still have fast disintegration. The orally disintegrating tablets according to the invention may comprise coloring agents. According to an embodiment of the invention, the orally disintegrating tablets may comprise color agents and whiteners such as FD&C-type dyes and lakes, fruit and vegetable extracts, titanium dioxide and combinations thereof.

### Example 2

### Preparation of orally disintegrating tablets using ready to use systems

Another way of preparing orally disintegrating tablets would be to use a ready to use system. Suitable for the purpose could be but not limited to: Pearlitol Flash (Roquette), Pharmaburst 500 (SPI Pharma), Ludiflash (BASF), ProSolv (JRS Pharma), ProSolv EasyTab (JRS Pharma), F-Melt (Fuji Chemical), SmartEx50 or SmartEx100 (Shin Etsu / Harke Pharma). These ready to use systems are co-processed systems where filler, disintegrant, glidant or similar are implemented in one powder mix. This saves handling of several excipients and ensures homogeneity between excipients.

In the present example five orally disintegrating tablets (ODT(g) - ODT(k)) with nicotine are prepared with ready to use systems in formulations as outlined in table 3A. The orally disintegrating tablets are prepared with NBT (nicotine bitartrate dihydrate).

In this example, the following conditions were applied. Punch used: 7.00 mm, circular, shallow concave, B tooling. Tablet weight: 100.0 mg.

**Table 3A - Orally disintegrating tablet compositions with different ready to use systems. Amounts are given in mg. ODT=Orally disintegrating tablet.**

| | **ODT(g)** | **ODT(h)** | **ODT(i)** | **ODT(j)** | **ODT(k)** |
|---|---|---|---|---|---|
| NBT | 2.85 | 2.85 | 2.85 | 2.85 | 2.85 |
| Ludiflash | 81.7 | - | - | - | - |
| Pearlitol Flash | - | 81.7 | - | - | - |
| SmartEx QD50 | - | - | 81.7 | - | - |
| F-Melt | - | - | - | 83.7 | - |
| ProSolv ODT G2 | - | - | - | - | 83.7 |
| Flavor | 3.05 | 3.05 | 3.05 | 3.05 | 3.05 |
| Sucralose | 0.4 | 0.4 | 0.4 | 0.4 | 0.4 |
| Sodium carbonate | 5.0 | 5.0 | 5.0 | 5.0 | 5.0 |
| Crospovidone | 5.0 | 5.0 | 5.0 | - | - |
| Croscarmellose Sodium | - | - | - | 3.0 | - |
| Sodium Starch Glycolate | - | - | - | - | 3.0 |
| Magnesium stearate | 2.0 | 2.0 | 2.0 | 2.0 | 2.0 |
| Total | 100.0 | 100.0 | 100.0 | 100.0 | 100.0 |

In the above examples a peppermint flavor in powder form was used. A mixture of peppermint and menthol may also be used. Of course, other flavors as described herein may be used as well, in combination with menthol and/or peppermint or replacing these. The flavor may be liquid or powdered or a combination, i.e. a liquid flavor and a powdered flavor is added.

Additionally, five orally disintegrating tables (ODT(l) - ODT(p)) with nicotine are prepared with ready to use systems in formulations as outlined in table 3B.

In this example, the following conditions were applied. Punch used: 7.00 mm, circular, shallow concave, B tooling. Tablet weight: 100.0 mg.

**Table 3B - Orally disintegrating tablet compositions with different ready to use systems and nicotine as nicotine bitartrate, NBT or nicotine polacrilex, NPR (15% nicotine load). Amounts are given in mg. ODT=Orally disintegrating tablet.**

| | **ODT (l)** | **ODT (m)** | **ODT (n)** | **ODT (o)** | **ODT (p)** |
|---|---|---|---|---|---|
| NBT | - | - | 3.0 | 3.0 | 3.0 |
| NPR | 6.7 | 6.7 | - | - | - |
| Ludiflash | 75.0 | - | - | - | - |
| Pearlitol Flash | - | 75.0 | - | - | - |
| SmartEx QD50 | - | - | 78.7 | - | - |
| F-Melt | - | - | - | 80.7 | - |
| ProSolv ODT G2 | - | - | - | - | 80.7 |
| Flavor | 5.9 | 5.9 | 5.9 | 5.9 | 5.9 |
| Sucralose | 0.4 | 0.4 | 0.4 | 0.4 | 0.4 |
| Sodium carbonate | 5.0 | 5.0 | 5.0 | 5.0 | 5.0 |
| Crospovidone | 5.0 | 5.0 | 5.0 | - | - |
| Croscarmellose Sodium | - | - | - | 3.0 | - |
| Sodium Starch Glycolate | - | - | - | - | 3.0 |
| Magnesium stearate | 2.0 | 2.0 | 2.0 | 2.0 | 2.0 |
| Total | 100.0 | 100.0 | 100.0 | 100.0 | 100.0 |

In the above examples a peppermint flavor in powder form was used. A mixture of peppermint and menthol may also be used. Of course, other flavors as described herein may be used as well, in combination with menthol and/or peppermint or replacing these. The flavor may be liquid or powdered or a combination, i.e. a liquid flavor and a powdered flavor is added.

Further four orally disintegrating tablets (ODT(1) - ODT(4)) with nicotine are prepared with varying amounts of MCC (microcrystalline cellulose) as filler, as outlined in table 3C. The orally disintegrating tablets are prepared with NBT (nicotine bitartrate dihydrate).

In this example, the following conditions were applied. Punch used: 7.00 mm, circular, shallow concave, B tooling. Tablet weight: 100.0 mg.

**Table 3C - Orally disintegrating tablet compositions with varying amounts of MCC. Amounts are given in mg. ODT=Orally disintegrating tablet.**

| | **ODT(1)** | **ODT(2)** | **ODT(3)** | **ODT(4)** |
|---|---|---|---|---|
| NBT | 3.0 | 3.0 | 3.0 | 3.0 |
| Microcrystalline cellulose | 0.0 | 5.0 | 10.0 | 20.0 |
| Mannitol | 79.7 | 74.7 | 69.7 | 59.7 |
| Crospovidone | 5.0 | 5.0 | 5.0 | 5.0 |
| Flavor | 5.9 | 5.9 | 5.9 | 5.9 |
| Sucralose | 0.4 | 0.4 | 0.4 | 0.4 |
| Sodium carbonate | 5.0 | 5.0 | 5.0 | 5.0 |
| Magnesium stearate | 1.0 | 1.0 | 1.0 | 1.0 |
| Total | 100.0 | 100.0 | 100.0 | 100.0 |

In the above examples a peppermint flavor in powder form was used. A mixture of peppermint and menthol may also be used. Of course, other flavors as described herein may be used as well, in combination with menthol and/or peppermint or replacing these. The flavor may be liquid or powdered or a combination, i.e. a liquid flavor and a powdered flavor is added.

Four orally disintegrating tablets, ODT(5) - ODT(8), with nicotine are prepared with varying amounts of disintegrant, as outlined in table 3D. The orally disintegrating tablets are prepared with NBT (nicotine bitartrate dihydrate).

In this example, the following conditions were applied. Punch used: 7.00 mm, circular, shallow concave, B tooling. Tablet weight: 100.0 mg.

**Table 3D - Orally disintegrating tablet compositions with varying amount of disintegrant. Amounts are given in mg. ODT=Orally disintegrating tablet.**

| | **ODT(5)** | **ODT(6)** | **ODT(7)** | **ODT(8)** |
|---|---|---|---|---|
| NBT | 3.0 | 3.0 | 3.0 | 3.0 |
| Mannitol | 41.7 | 39.2 | 41.7 | 31.7 |
| Microcrystalline cellulose | 43 | 43 | 43 | 43 |
| Crospovidone | 0.0 | 2.5 | 5.0- | 10.0 |
| Flavor | 5.9 | 5.9 | 5.9 | 5.9 |
| Sucralose | 0.4 | 0.4 | 0.4 | 0.4 |
| Sodium carbonate | 5.0 | 5.0 | 5.0 | 5.0 |
| Magnesium stearate | 1.0 | 1.0 | 1.0 | 1.0 |
| Total | 100.0 | 100.0 | 100.0 | 100.0 |

In the above examples a peppermint flavor in powder form was used. A mixture of peppermint and menthol may also be used. Of course, other flavors as described herein may be used as well, in combination with menthol and/or peppermint or replacing these. The flavor may be liquid or powdered or a combination, i.e. a liquid flavor and a powdered flavor is added.

Three orally disintegrating tablets, ODT(9) - ODT(11), with nicotine are prepared with varying types of lubricants, as outlined in table 3E. The orally disintegrating tablets are prepared with NBT (nicotine bitartrate dihydrate).

In this example, the following conditions were applied. Punch used: 7.00 mm, circular, shallow concave, B tooling. Tablet weight: 100.0 mg.

**Table 3E - Orally disintegrating tablet compositions. Amounts are given in mg. ODT=Orally disintegrating tablet.**

| | **ODT (9)** | **ODT (10)** | **ODT (11)** |
|---|---|---|---|
| NBT | 3.0 | 3.0 | 3.0 |
| Microcrystalline cellulose | 5 | 5 | 5 |
| Mannitol | 81 | 80 | 80 |
| Crospovidone | 5.0 | 5.0 | 5.0 |
| Sodium carbonate | 5.0 | 5.0 | 5.0 |
| Magnesium stearate | 1.0 | - | - |
| Sodium stearyl fumarate | - | 2.0 | - |
| Compritol HD5 | - | - | 2.0 |
| Total | 100.0 | 100.0 | 100.0 |

Three orally disintegrating tablets, ODT(12) - ODT(14), with nicotine are prepared, as outlined in table 3F. The orally disintegrating tablets are prepared with NBT (nicotine bitartrate dihydrate).

In this example, the following conditions were applied. Punch used: 7.00 mm, circular, shallow concave, B tooling. Tablet weight: 75.0 mg.

**Table 3F - Orally disintegrating tablet compositions. Amounts are given in mg. ODT=Orally disintegrating tablet. ODT(13) was made similar to ODT(12) but without buffer. ODT(14) was made similar to ODT(12) but without disintegrant.**

| | **ODT(12)** | **ODT(13)** | **ODT(14)** |
|---|---|---|---|
| SmartEx QD 50 | 60.0 | 65.0 | 65.0 |
| NBT | 3.0 | 3.0 | 3.0 |
| Sodium carbonate anhydrous | 5.0 | 0.0 | 5.0 |
| Crospovidone (Kollidon CL-F, BASF) | 5.0 | 5.0 | 0.0 |
| Flavor | 0.8 | 0.8 | 0.8 |
| Aerosil 200 (silicium dioxide) | 0.2 | 0.2 | 0.2 |
| Magnesium Stearate | 1.0 | 1.0 | 1.0 |
| Total | 75.0 | 75.0 | 75.0 |

In the above examples a peppermint flavor in powder form was used. A mixture of peppermint and menthol may also be used. Of course, other flavors as described herein may be used as well, in combination with menthol and/or peppermint or replacing these. The flavor may be liquid or powdered or a combination, i.e. a liquid flavor and a powdered flavor is added.

ODT(12)-ODT(13) were pressed to a hardness of 15-20 N. ODT (14) was pressed to a hardness of 25-35 N.

### Example 3

### Preparation of orally disintegrating tablets

Orally disintegrating tablets (ODT) with 1, 2, and 3 mg nicotine, respectively, are prepared with formulations as outlined in table 3G. The orally disintegrating tablets are prepared with NBT (nicotine bitartrate dihydrate with a nicotine content of 32.56%). Punch used: 7.00 mm, circular, shallow concave, D tooling. Tablet weight: 100.0 mg.

**Table 3G - Orally disintegrating tablet compositions. Amounts are given in mg. ODT=Orally disintegrating tablet. HIS = high intensity sweetener.**

| | **ODT (21)** | **ODT (22)** | **ODT (23)** | **ODT (24)** | **ODT (25)** | **ODT (26)** |
|---|---|---|---|---|---|---|
| NBT | 3.07 | 6.14 | 9.21 | 9.21 | 9.21 | 9.21 |
| Mannitol | 77.58 | 72.36 | 66.79 | 64.29 | 67.29 | 70.29 |
| Microcrystalline cellulose | 5.0 | 5.0 | 5.0 | 10.0 | 10.0 | 10.0 |
| Sodium carbonate | 2.85 | 5.0 | 7.5 | 5.0 | 5.0 | 5.0 |
| Crospovidone | 8.0 | 8.0 | 8.0 | 8.0 | 5.0 | 2.0 |
| Flavor | 2.0 | 2.0 | 2.0 | 2.0 | 2.0 | 2.0 |
| HIS | 0.5 | 0.5 | 0.5 | 0.5 | 0.5 | 0.5 |
| Sodium stearyl fumarate | 1.0 | 1.0 | 1.0 | 1.0 | 1.0 | 1.0 |
| Total | 100.0 | 100.0 | 100.0 | 100.0 | 100.0 | 100.0 |

For example peppermint and/or menthol may be used as flavors. Other flavors are also usable in ODT(21)-ODT(26).

Sucralose may be used as high intensity sweetener. Other high intensity sweeteners may also be used.

### Example 4

### Preparation of orally disintegrating tablets

Orally disintegrating tablets (ODT) with 2 mg nicotine are prepared with formulations as outlined in table 3H. The orally disintegrating tablets are prepared with NBT (nicotine bitartrate dihydrate). Punch used: 7.00 mm, circular, shallow concave, D tooling. Tablet weight: 100.0 mg.

**Table 3H - Orally disintegrating tablet compositions. Amounts are given in mg. ODT=Orally disintegrating tablet. HIS = high intensity sweetener.**

| | **ODT (31)** | **ODT (32)** | **ODT (33)** | **ODT (34)** |
|---|---|---|---|---|
| Sorbitol | 74.61 | - | - | - |
| Isomalt | - | 74.61 | - | - |
| Xylitol | - | - | 74.61 | - |
| Mannitol | - | - | - | 74.61 |
| NBT | 6.14 | 6.14 | 6.14 | 6.14 |
| Microcrystalline cellulose | 2.1 | 2.1 | 2.1 | 2.1 |
| Guar gum | 0.4 | 0.4 | 0.4 | 0.4 |
| Sodium carbonate | 5.0 | 5.0 | 5.0 | 5.0 |
| Crospovidone | 8.0 | 8.0 | 8.0 | 8.0 |
| Flavor | 2.0 | 2.0 | 2.0 | 2.0 |
| HIS | 0.75 | 0.75 | 0.75 | 0.75 |
| Sodium stearyl fumerate | 1.0 | 1.0 | 1.0 | 1.0 |
| Total | 100.0 | 100.0 | 100.0 | 100.0 |

For example peppermint and/menthol may be used as flavors. Other flavors are also usable in ODT(31)-ODT(34).

Sucralose may be used as high intensity sweetener. Other high intensity sweeteners may also be used.

### Example 5

### In vitro disintegration of orally disintegrating tablets

The in vitro disintegration of the orally disintegrating tablets of example 1 and 2 was carried out in accordance to European Pharmacopeia 9.0, section 2.9.1, *Disintegration of tablets and capsules.*

The results for example 1 are outlined in table 4.

**Table 4 - In vitro disintegration, hardness, friability. Time is given in seconds.**

| | **Mean in vitro disintegration (sec)** | **Mean hardness (N)** | **Mean friability (%)** |
|---|---|---|---|
| ODT(a) | 21 | 14 | 0.3 |
| ODT(b) | 23 | 12 | 0.6 |
| ODT(c) | 29 | 14 | 0.5 |
| ODT(d) | 15 | 19 | 0.0 |
| ODT(e) | 13 | 15 | 0.2 |
| ODT(f) | 11 | 14 | 0.2 |

The results for example 2 are outlined in table 5.

**Table 5 - In vitro disintegration, hardness, friability. Time is given in seconds.**

| | **Mean in vitro disintegration (sec)** | **Mean hardness (N)** | **Mean friability (%)** |
|---|---|---|---|
| ODT(h) | 40 | 16 | 0.8 |
| ODT(i) | 10 | 17 | 0.3 |
| ODT(j) | 42 | 17 | 1.0 |
| ODT(k) | 45 | 17 | 0.9 |

### Example 6 - Evaluation of orally disintegrating tablets - burning

Evaluation of burning sensation is performed as described in the following.

Nicotine burning was evaluated by a test panel of 6 trained assessors. After calibration by means of chewing two standard nicotine containing chewing gums with "known" burning intensity, each assessor evaluates the burning sensation in the throat on a scale from 0 to 15, where 0 is no burning at all and 15 is the most intense burning. Each assessor evaluates all samples twice. The evaluations are noted for the time periods indicated. Average values are calculated.

Burning was evaluated for ODT(21), both administered on the tongue and between the upper lip and the gum. ODT(21) was evaluated to give an average burning score of 5.1 in the range between 1.5 and 5 minutes when administered on the tongue. In the same time range, the same tablet, ODT(21), was given an average burning score of 0.0 when administered between the upper lip and the gum.

Thus, the administration between the lip and gum was observed to give a significant reduction of burning to a point of below the minimum threshold of sensing any burning.

### Example 7 - Nicotine effect

Nicotine effect was evaluated using a panel of three users evaluating all samples twice.

Each user assessed the nicotine effect, i.e. the perceived effect of nicotine stimulating the body on a scale from 0 to 5, where 0 is no effect at all and 5 is the most intense nicotine effect perceived. The average times for ODT(21) is indicated in tables 6A-6B.

**Table 6A. Perceived nicotine effect. Under lip refers to the position between the upper lip and gum.**

| **Time [min]** | **1** | **1.5** | **2** | **2.5** | **3** | **4.5** |
|---|---|---|---|---|---|---|
| | **Evaluated nicotine effect** | | | | | |
| Under lip | 0 | 1 | 1.5 | 2 | 2.5 | 4 |
| On tongue | 0.5 | 1 | 1 | 1.5 | 1.5 | 2 |

**Table 6B. Perceived nicotine effect. Under lip refers to the position between the upper lip and gum.**

| **Time [min]** | **5** | **5.5** | **6** | **7** | **8** | **10** | **12** |
|---|---|---|---|---|---|---|---|
| | **Evaluated nicotine effect** | | | | | | |
| Under lip | 4 | 4 | 4.5 | 4.5 | 4.5 | 4 | 3 |
| On tongue | 2 | 2.5 | 2 | 2 | 2 | 1.5 | 1 |

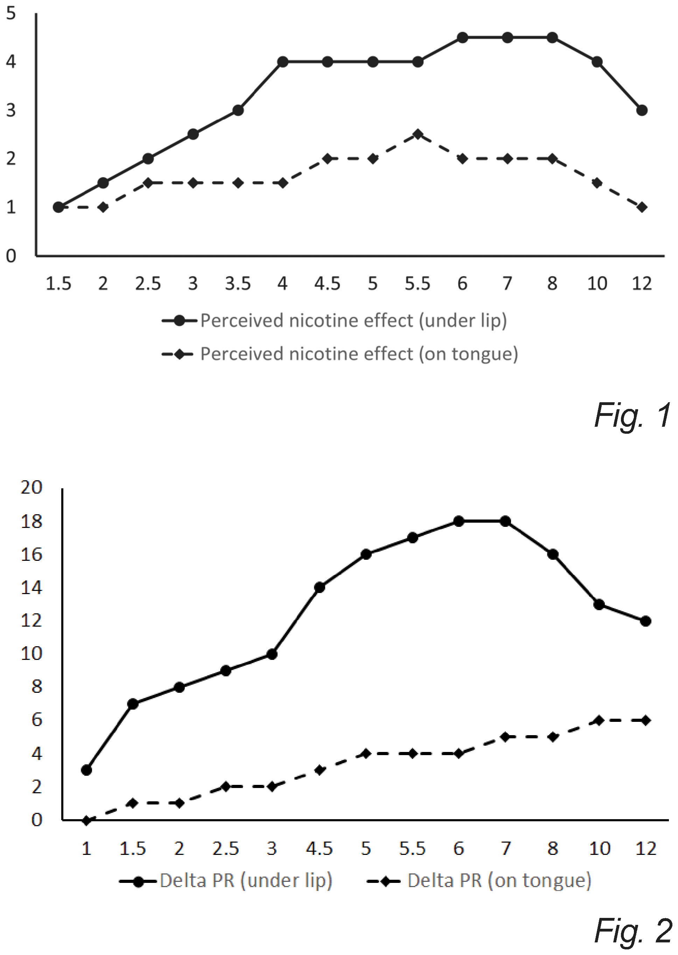

As observed from tables 6A-6B and shown in figure 1, the administration between the upper lip and gum gave a significant increase in perceived effect compared to administration on the tongue within the evaluated time range of 1-12 minutes.

Also, as an alternative measure of the nicotine effect, the difference in pulse rate was noted relative to the pulse rate at rest before administration. Higher difference in pulse rate thus corresponds to higher effect of nicotine. Difference in pulse rates is noted in below tables 7A-7B.

**Table 7A. Difference in pulse rate. Under lip refers to the position between the upper lip and gum.**

| **Time [min]** | **1.5** | **2** | **2.5** | **3** | **3.5** | **4** | **4.5** |
|---|---|---|---|---|---|---|---|
| | **Difference in pulse rate (Delta PR)** | | | | | | |
| Under lip | 7 | 8 | 9 | 10 | 10 | 14 | 14 |
| On tongue | 1 | 1 | 2 | 2 | 3 | 3 | 3 |

**Table 7B. Difference in pulse rate. Under lip refers to the position between the upper lip and gum.**

| **Time [min]** | **5** | **5.5** | **6** | **7** | **8** | **10** | **12** |
|---|---|---|---|---|---|---|---|
| | **Difference in pulse rate (Delta PR)** | | | | | | |
| Under lip | 16 | 17 | 18 | 18 | 16 | 13 | 12 |
| On tongue | 4 | 4 | 4 | 5 | 5 | 6 | 6 |

As observed from tables 7A-7B and shown in figure 2, the administration between the upper lip and gum gave a significant increase in effect compared to administration on the tongue as measured by the increase in pulse rate, within the evaluated time range of 1-12 minutes.

### Example 8 - Nicotine effect

Several orally disintegrating nicotine tablets were tested and compared with commercially available nicotine products by measuring nicotine plasma concentrations over time in a clinical trial.

24 healthy subjects were selected.

A single dose of the relevant nicotine product was administered to each subject and blood samples were taken at suitable time periods extending from the time of administration (t=0).

In table 8, average nicotine plasma concentrations over the first 60 minutes from oral administration are shown for ODT(21)-ODT(23)) administered between the gum and the upper lip according to the invention as well as conventionally on the tongue or sublingually, and for a single dispensing of Nicorette Quickmist (a commercially available nicotine mouth spray product used as a reference in the trial).

**Table 8. Nicotine plasma concentration for various nicotine products over time from administration.**

| **Time [min]** | **Nicorette QuickMist (2 mg)** | **ODT (22) on tongue** | **ODT (22) under tongue** | **ODT(21) gum-lip** | **ODT(22) gum-lip** | **ODT(23) gum-lip** |
|---|---|---|---|---|---|---|
| 0 | 0.0 | 0.0 | 0.0 | 0.0 | 0.0 | 0.0 |
| 4 | 4.4 | 3.8 | 4.2 | 1.3 | 1.8 | 3.8 |
| 8 | 6.6 | 6.7 | 7.8 | 4.1 | 10.2 | 13.9 |
| 12 | 6.4 | 6.6 | 7.5 | 4.9 | 11.2 | 15.2 |
| 16 | 6.6 | 6.4 | 6.9 | 4.9 | 10 | 13.6 |
| 20 | 6.6 | 6.3 | 6.5 | 4.7 | 9.7 | 13.1 |
| 25 | 6 | 5.9 | 6.4 | 4.3 | 8.8 | 12.1 |
| 30 | 5.9 | 5.7 | 6.3 | 4.2 | 8.5 | 11.4 |
| 35 | 5.5 | 5.5 | 6.1 | 4 | 8.1 | 11 |
| 40 | 5.3 | 5.4 | 5.9 | 3.8 | 7.6 | 10.6 |
| 60 | 4.6 | 5 | 5.3 | 3.2 | 6.4 | 9.2 |

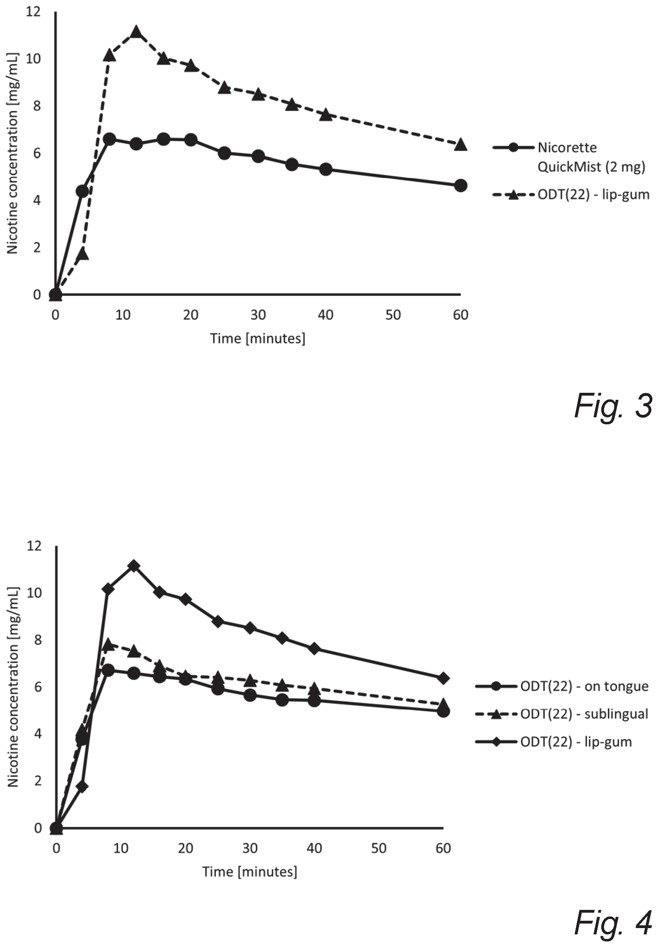

A comparison of the nicotine plasma concentration resulting from ODT(22) administered between lip and gum and of the Quickmist reference product is shown in figure 3.

As can be seen in figure 3, the inventive administration of ODT(22) significantly increases plasma concentration, compared to the reference, of nicotine from 8 minutes after administration to 60 minutes after administration, i.e. for the remaining time points.

A comparison of the nicotine plasma concentration resulting from ODT(22) administered between upper lip and gum and the same ODT(22) administered sublingually (under the tongue) and on the tongue respectively is shown in figure 4.

As can be seen in figure 4, sublingual administration appears to result in slightly higher nicotine plasma concentration compared to administration on the tongue. However, the increase in nicotine plasma concentration is significantly increased when administering the same tablet between lip and gum. Thus, the inventive administration between lip and gum clearly leads to an increase in nicotine plasma concentration compared to administration on the tongue that far exceeds the improvement from administering the ODT sublingually. This finding was highly unexpected, as sublingual administration is typically considered a preferred route of oral administration in terms of fast and effective uptake of nicotine.

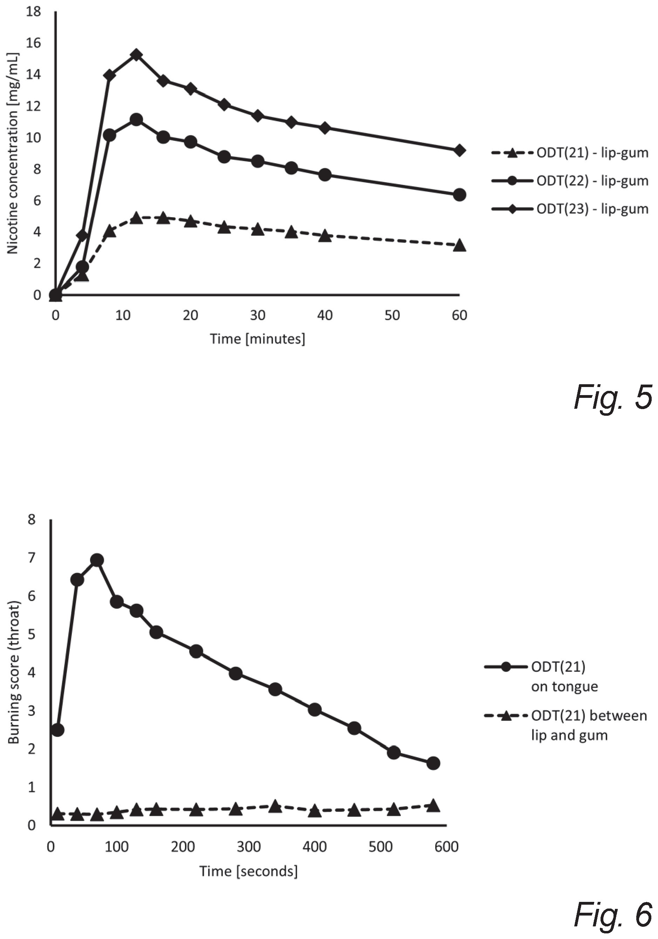

A comparison of the nicotine plasma concentration resulting from ODT(21), ODT(22), and ODT(23), all administered between upper lip and gum is shown in figure 5.

As can be seen in figure 5, the obtained nicotine plasma concentration correlates to the total nicotine dose of the ODT, as ODT(21) comprises 1 mg nicotine, ODT(22) comprises 2 mg nicotine, and ODT(23) comprises 3 nicotine. As can be observed, the increase in nicotine plasma concentration when increasing from 2 mg to 3 mg is somewhat comparable to the increase when increasing from 1 mg to 2 mg. This finding was unexpected, as increasing the nicotine dose in nicotine delivery products are often found to provide a less efficient change in nicotine plasma concentration.

**Table 9. Pharmacokinetic parameters. The AUC area under the curve is obtained by using measurements for all available time points in the clinical trial.**

| | Cmax [ng/mL] | AUC [h*ng/mL] | Tmax [min] | Disintegration time, in vivo |
|---|---|---|---|---|
| Nicorette QuickMist (2 mg) | 8.2 | 18.9 | 16 | N/A |
| ODT (22) on tongue | 8.4 | 20.2 | 20 | 37 s |
| ODT (22) under tongue (sublingual) | 8.9 | 21.0 | 12 | 64 s |
| ODT(21) gum-lip | 5.7 | 13.3 | 16 | 4 min |
| ODT(22) gum-lip | 12.8 | 25.6 | 12 | 4 min |
| ODT(23) gum-lip | 17.6 | 36.7 | 12 | 4 min |

As shown in table 9, the maximum nicotine plasma concentration (Cmax) of ODT(22) was significantly higher when administered between the gum and lip compared to a conventional administration on the tongue or administration under the tongue.

Similarly, the area under the curve (AUC) for ODT(22) was significantly higher when administered between the gum and lip compared to a conventional administration on the tongue or administration under the tongue.

Also, comparing the maximum nicotine plasma concentration (Cmax) and the area under the curve (AUC) for the comparable ODT(21)-ODT(23) shows that gradually increasing the dose of nicotine from 1 mg (ODT(21)) to 2 mg (ODT(22)) and then to 3 mg (ODT(23)) results in gradually increase in both Cmax and AUC consistent with the direct observations of figure 5.

Finally, the values of table 9 demonstrates how the inventive administration results in a shorter Tmax, indicating a faster nicotine craving relief.

### Example 9 - Evaluation of urge to smoke

In connection with the clinical trial described in example 8, the subjects in the trial evaluated perceived desire to smoke for the inventive administration of ODT(22) as well as comparative administration of ODT(22) on the tongue, sublingual administration of ODT(22), and the commercially available Quickmist mouth spray. Desire to smoke was evaluated on a scale between 0 (no desire to smoke) and 100 (strongest desire to smoke).

At a time period of 25 minutes after administration the desire to smoke was evaluated. The desire to smoke values were compared to baseline values determined before administration. Results of the reduction in desire to smoke are listed in below table 10.

**Table 10. Reduction in desire to smoke evaluated 25 minutes after administration of the relevant 2 mg nicotine product.**

| Nicotine product | ODT(22) lip-gum | ODT(22) on tongue | ODT(22) sublingual | QuickMist |
|---|---|---|---|---|
| Desire to smoke | 43 | 33 | 37 | 32 |

As can be seen from table 10, a significant reduction in desire to smoke was obtained for the inventive lip-gum administration of ODT(22), exceeding reduction in desire to smoke for corresponding administration on the tongue and sublingual administration. Also, a significantly improved reduction of desire to smoke was observed compared to Quickmist.

### Example 10 - Evaluation of burning sensation in the throat

Nicotine burning was evaluated by a test panel of 8 trained assessors. At first calibration of nicotine burning was made by means of placing a representative standard nicotine tablet in the mouth, on the tongue and sucking it to complete disintegration. Then, nicotine burning at fixed time points 30 and 180 seconds was calibrated across the test panel. In the test, each assessor evaluated the burning sensation in the throat on a scale from 0 to 15, where 15 is the most intense burning. Each assessor evaluated all samples twice. The evaluations were noted for the time points indicated. Average values of all assessors were calculated. Results are given in below table 11 and are also shown in figure 6.

**Table 11. Burning score in throat as a function of time in seconds after administration**

| | **ODT(21) on tongue** | **ODT(21) lip-gum** |
|---|---|---|
| Time [seconds] | Burning score | |
| 10 | 2.5 | 0.3 |
| 40 | 6.4 | 0.3 |
| 70 | 6.9 | 0.3 |
| 100 | 5.9 | 0.4 |
| 130 | 5.6 | 0.4 |
| 160 | 5.1 | 0.4 |
| 220 | 4.6 | 0.4 |
| 280 | 4.0 | 0.4 |
| 340 | 3.6 | 0.5 |
| 400 | 3.0 | 0.4 |
| 460 | 2.5 | 0.4 |
| 520 | 1.9 | 0.4 |
| 580 | 1.6 | 0.5 |

Conclusion: The sensory test demonstrates that administration of the orally disintegrating nicotine tablets between the gum and the lip unexpectedly leads to a significantly lower level of throat burning compared to conventional administration in the oral cavity on the tongue.

In fact, the level of burning resulting from the administration between the lip and the gum is almost eliminated compared to administration on the tongue.

This demonstrates that administration of orally disintegrating nicotine tablets between the lip and gum unexpectedly results in a combination of a very significant reduction in the undesirable side effect of nicotine throat burning, while at the same time the absorption of nicotine is significantly improved, as shown in above example 8.

## Claims

1. An orally disintegrating nicotine tablet for use in alleviation of nicotine craving, the tablet comprising nicotine and a pH regulating agent, wherein the tablet is administered between the lip and gum.

2. The orally disintegrating nicotine tablet according to claim 1, wherein the tablet is administered between the upper lip and gum.

3. The orally disintegrating nicotine tablet according to claim 1 or 2, wherein the tablet is designed to disintegrate within a period of 300 seconds upon oral administration between the lip and gum, such as within 270 seconds upon oral administration between the lip and gum, such as within 240 seconds upon oral administration, such as within a period of 210 seconds upon oral administration, such as within a period of 180 seconds upon oral administration, such as within a period of 150 seconds upon oral administration, such as within a period of 120 seconds upon oral administration, such as within a period of 90 seconds upon oral administration, such as within a period of 60 seconds upon oral administration.

4. The orally disintegrating nicotine tablet according to any of claims 1-3, wherein the tablet is designed to disintegrate within a period of 60 seconds in vitro, such as within 50 seconds in vitro, such as within 40 seconds in vitro.

5. The orally disintegrating nicotine tablet according to any of claims 1-4, wherein the tablet is a compressed orally disintegrating nicotine tablet.

6. The orally disintegrating nicotine tablet according to any of claims 1-5, wherein the tablet comprises at least one sugar alcohol.

7. The orally disintegrating nicotine tablet according to claim 6, wherein the tablet comprises the at least one sugar alcohol sugar alcohol in an amount of at least 40% by weight of the tablet, such as at least 50% by weight of the tablet, such as at least 60% by weight of the tablet, such as at least 70% by weight of the tablet, such as at least 80% by weight of the tablet.

8. The orally disintegrating nicotine tablet according to claim 6 or 7, wherein the at least one sugar alcohol comprises sugar alcohol selected from the group consisting of xylitol, maltitol, mannitol, erythritol, isomalt, sorbitol, lactitol and any combination thereof.

9. The orally disintegrating nicotine tablet according to any of claims 1-8, wherein the tablet further comprises a disintegrant.

10. The orally disintegrating nicotine tablet according to any of claims 1-9, wherein the tablet comprises disintegrant in an amount of at least 0.5% by weight of the tablet, such as at least 1% by weight of the tablet, such as at least 2% by weight of the tablet, such as at least 3% by weight of the tablet, such as at least 5% by weight of the tablet.

11. The orally disintegrating nicotine tablet according to claim 9 or 10, wherein the disintegrant is selected from the list consisting of starch, pregelatinated starch, modified cellulose, alginates, ion-exchange resin, calcium silicate, crosslinked cellulose, crosslinked polyvinyl pyrrolidone, crosslinked starch, crosslinked alginic acid, and combinations thereof.

12. The orally disintegrating nicotine tablet according to any of claims 1-11, wherein the disintegrant comprises a superdisintegrant.

13. The orally disintegrating nicotine tablet according to any of claims 1-11, wherein the tablet comprises superdisintegrant in an amount of at least 0.5% by weight of the tablet, such as at least 1% by weight of the tablet, such as at least 2% by weight of the tablet, such as at least 3% by weight of the tablet, such as at least 5% by weight of the tablet.

14. The orally disintegrating nicotine tablet according to any of claims 12-13, wherein the superdisintegrant is selected from the group consisting of crosslinked cellulose, crosslinked polyvinyl pyrrolidone, crosslinked starch, crosslinked alginic acid, and any combinations thereof.

15. The orally disintegrating nicotine tablet according to any of claims 1-14, wherein the tablet is compressed using a compression force of 1 to 35 kN, such as 2 to 30 kN, such as 2 - 20 kN.

16. The orally disintegrating nicotine tablet according to any of claims 1-15, wherein the nicotine is selected from the group consisting of a nicotine salt, nicotine free base, a nicotine-ion exchange resin combination, a nicotine inclusion complex or nicotine in any non-covalent binding, nicotine bound to zeolites, nicotine bound to cellulose, nicotine bound to starch microspheres, and any combination thereof.

17. The orally disintegrating nicotine tablet according to any of claims 1-16, wherein the nicotine comprises nicotine salt.

18. The orally disintegrating nicotine tablet according to claim 17, wherein the nicotine salt is selected from the group consisting of nicotine ascorbate, nicotine aspartate, nicotine benzoate, nicotine monotartrate, nicotine bitartrate, nicotine chloride, nicotine citrate, nicotine fumarate, nicotine lactate, nicotine mucate, nicotine laurate, nicotine levulinate, nicotine malate, nicotine perchlorate, nicotine pyruvate, nicotine salicylate, nicotine sorbate, nicotine succinate, nicotine sulfate, and any combination thereof.

19. The orally disintegrating nicotine tablet according to any of claims 1-18, wherein the pH regulating agent is an alkaline pH regulating agent, such as an alkaline buffering agent.

20. The orally disintegrating nicotine tablet according to any of claims 1-19, wherein the pH regulating agent comprises pH regulating agent selected from the group consisting of sodium carbonate, sodium bicarbonate, potassium carbonate, potassium bicarbonate, trometamol, amino acids, di-alkali hydrogen phosphate, tri-alkali phosphate, or any combination thereof.

21. The orally disintegrating nicotine tablet according to any of claims 1-20, wherein the tablet has a weight of no more than 600 mg, such as no more than 500 mg, such as no more than 400 mg, such as no more than 300 mg, such as no more than 200 mg, such as no more than 150 mg, such as no more than 120 mg, such as no more than 100 mg.

22. The orally disintegrating nicotine tablet according to any of claims 1-21, wherein the tablet is a one-layer tablet.

23. The orally disintegrating nicotine tablet according to any of claims 1-22, wherein the area under the curve (AUC) for nicotine is increased by administration between the lip and gum compared to administration on the tongue.

24. The orally disintegrating nicotine tablet according to any of claims 1-23, wherein the area under the curve (AUC) for nicotine is increased by administration between the lip and gum compared to sublingual administration.
